# EUROPEAN PATENT APPLICATION

(11) **EP 4 497 419 A1**
(43) Date of publication of application: **29.01.2025**
(21) Application number: 24183790.5
(22) Date of filing: 21.06.2024
(51) Int. Cl.: A61F 2/95

(54) **LOADING DEVICES FOR LOADING A STENT-GRAFT INTO A GRAFT COVER**

(30) Priority: 28.07.2023 US 202363516414 P; 02.04.2024 US 202418624856
(71) Applicant: Medtronic Vascular, Inc., Santa Rosa, CA 95403 (US)
(72) Inventor: MONGILIO, Kerry Page, Santa Rosa, 95403 (US); BROWN, Alyssa, Santa Rosa, 95403 (US); SETHNA, Sohrab, Santa Rosa, 95403 (US)
(74) Representative: Zimmermann & Partner Patentanwälte mbB

(57) **Abstract**

A loading device for loading a graft-stent into a graft cover includes an eye shaft, an adjustment shaft, a rod, and a collar. The eye shaft includes an eyelet disposed at a first end of the eye shaft. The adjustment shaft includes a first portion adjustably coupled to a second end of the eye shaft opposite the first end of the eye shaft such that an overall length of the loading device may be lengthened or shortened via the adjustment shaft. The rod is coupled to a second portion of the adjustment shaft opposite the first portion of the adjustment shaft. The collar is disposed over the rod and includes a collar lumen configured to receive a suture between an inner surface of the collar and an outer surface of the rod.

## Description

### CROSS-REFERENCE TO RELATED APPLICATIONS

This application claims the benefit of U.S. Provisional Patent Application Serial No. 63/516,414, filed July 28, 2023, the entire content of which is incorporated herein by reference.

### TECHNICAL FIELD

The present discloses relates to devices and methods for loading a stent-graft into a graft cover.

### BACKGROUND

Transcatheter stent-grafts need to be loaded into a delivery device before procedural use. Under most scenarios, the loading process occurs at the manufacturing site of the stent-graft and the delivery device. However, there are scenarios where the stent-graft may need to be loaded into the delivery device at the geographic location of the procedure (*e*.*g*., a hospital or other treatment facility).

Many current loading devices for loading stent-grafts into delivery devices are designed to be used with stent-grafts that have externally sewn stent rings, exposed stent nodes, crowns, or peaks, or have been permanently modified to include suture loops for the sole purpose of loading. For stent-grafts with internally sewn stent rings, the use of existing loading devices is difficult and can damage the stent-graft. Existing tools and loading devices are difficult to attach and detach from these stent-grafts, and the increased interaction between existing loading devices and the internally sewn stent ring can lead to stent ring damage and can increase the loading force on the stent-graft during the loading process, potentially compromising the integrity of the stent-graft.

Accordingly, there is a need for improved loading devices and methods for loading stent-grafts into graft covers.

### SUMMARY

In an example, the present disclosure relates to a loading device for loading a graft-stent into a graft cover, the loading device including: an eye shaft including an eyelet disposed at a first end of the eye shaft; an adjustment shaft, wherein a first portion of the adjustment shaft is adjustably coupled to a second end of the eye shaft opposite the first end of the eye shaft such that an overall length of the loading device may be lengthened or shortened via the adjustment shaft; and a rod coupled to a second portion of the adjustment shaft opposite the first portion of the adjustment shaft.

In another example hereof, the loading device according to any of the preceding or following examples further includes a collar disposed over the rod, the collar including a collar lumen, wherein the collar lumen is configured to receive a suture between an inner surface of the collar and an outer surface of the rod.

In another example hereof, in the loading device according to any of the preceding or following examples, the eyelet includes a gap configured to receive a suture therethrough.

In another example hereof, in the loading device according to any of the preceding or following examples, the second end of the eye shaft includes a first threaded portion, and the first portion of the adjustment shaft includes a second threaded portion configured to engage the first threaded portion to adjustably couple the adjustment shaft and the eye shaft.

In another example hereof, in the loading device according to any of the preceding or following examples, the eye shaft includes a cavity at the second end, wherein the first threaded portion is disposed within the cavity, and wherein the first portion of the adjustment shaft is configured to be received within the cavity such that the second threaded portion engages the first threaded portion to adjustably couple the adjustment shaft and the eye shaft.

In another example hereof, in the loading device according to any of the preceding or following examples, the second portion of the adjustment shaft includes a rod cavity, wherein the rod is configured to be disposed in the rod cavity to couple the rod to the adjustment shaft.

In another example hereof, in the loading device according to any of the preceding or following examples, the rod is configured to be press fit into the rod cavity to couple the rod to the adjustment shaft.

In another example hereof, the loading device according to any of the preceding or following examples further includes: a stent-graft including a stent coupled to a graft material, the stent including a plurality of struts coupled to each other at crowns; and a suture, wherein the suture is configured to be threaded through a plurality of the crowns at a first end of the stent-graft and to the eyelet of the eye shaft, and wherein lengthening the overall length of the loading device via the adjustment shaft and the eye shaft is configured to make the suture taut and to radially converge the crowns at the first end of the stent-graft.

In another example hereof, in the loading device according to any of the preceding or following examples, a first end and a second end of the suture are coupled to each other adjacent the eyelet, and wherein a middle portion of the suture is threaded through the crowns at the first end of the stent.

In another example hereof, in the loading device according to any of the preceding or following examples, the stent includes a plurality of stent rings, wherein the suture is coupled to the crowns of one of the plurality of stent rings at the first end of the stent-graft.

In another example hereof, in the loading device according to any of the preceding or following examples, the suture includes a plurality of sutures.

In another example hereof, the present disclosure relates to a loading device for loading a graft-stent into a graft cover, the loading device including: a proximal shaft having a first eyelet disposed at a proximal end of the proximal shaft; a distal eye shaft including a second eyelet disposed at a distal end of the distal eye shaft; a distal adjustment shaft, wherein a distal portion of the distal adjustment shaft is adjustably coupled to a proximal end of the distal eye shaft opposite the distal end of the eye shaft such that an overall length of the loading device may be lengthened or shortened via the distal adjustment shaft; and a rod having a proximal portion coupled to a distal end of the proximal shaft and a distal portion coupled to a proximal portion of the distal adjustment shaft.

In another example hereof, in the loading device according to any of the preceding or following examples, the distal eyelet includes a gap configured to receive a suture therethrough.

In another example hereof, in the loading device according to any of the preceding or following examples, the proximal end of the eye shaft includes a first threaded portion and the distal portion of the adjustment shaft includes a second threaded portion configured to engage the first threaded portion to adjustably couple the distal adjustment shaft and the distal eye shaft.

In another example hereof, in the loading device according to any of the preceding or following examples, the distal eye shaft includes a cavity at the proximal end thereof, wherein the first threaded portion is disposed within the cavity, and wherein the distal portion of the adjustment shaft is configured to be received within the cavity such that the second threaded portion engages the first threaded portion to adjustably couple the distal adjustment shaft and the distal eye shaft.

In another example hereof, in the loading device according to any of the preceding or following examples, the proximal portion of the distal adjustment shaft includes a first rod cavity, wherein the distal portion of the rod is configured to be disposed in the first rod cavity to couple the rod to the distal adjustment shaft, and wherein the distal end of the proximal shaft include a second rod cavity, wherein the proximal portion of the rod is configured to be disposed in the second rod cavity to couple the rod to the proximal shaft.

In another example hereof, in the loading device according to any of the preceding or following examples, the distal portion of the rod is configured to be press fit into the first rod cavity to couple the rod to the distal adjustment shaft, and the proximal portion of the rod is configured to be press fit into the second rod cavity to couple the rod to the proximal shaft.

In another example hereof, the loading device according to any of the preceding or following examples further includes: a stent-graft including a stent coupled to a graft material, the stent including a plurality of struts coupled to each other at crowns; and a suture, wherein the suture is configured to be threaded through a plurality of the crowns at a proximal end of the stent-graft, extend proximally to the first eyelet, loop through the first eyelet, extend distally past the stent-graft and to the second eyelet at the distal end of the distal eye shaft, and wherein lengthening the overall length of the loading device via the distal adjustment shaft and the distal eye shaft is configured to make the suture taut and to radially converge the crowns at the proximal end of the stent.

In another example hereof, in the loading device according to any of the preceding or following examples, a first end and a second end of the suture are coupled to each other adjacent the second eyelet, and wherein a middle portion of the suture is threaded through the crowns at the proximal end of the stent-graft.

In another example hereof, in the loading device according to any of the preceding or following examples, the stent includes a plurality of stent rings, wherein the suture is coupled to the crowns of one of the plurality of stent rings at the proximal end of the stent-graft.

In another example hereof, in the loading device according to any of the preceding or following examples, the suture includes a plurality of sutures.

In another example hereof, in the loading device according to any of the preceding or following examples, the proximal shaft includes a proximal eye shaft and a proximal adjustment shaft adjustably coupled to each other to lengthen or shorten the overall length of the loading device.

In another example hereof, the present disclosure relates to a method of loading a stent-graft into a graft cover including: coupling a suture to a first end of a stent-graft in a radially expanded configuration by weaving the suture through adjacent crowns of a first stent ring at the first end of the stent-graft; advancing a first end and a second end to of the suture through a collar of a loading device, the loading device including an eye shaft including an eyelet disposed at a first end of the eye shaft; an adjustment shaft, wherein a first portion of the adjustment shaft is adjustably coupled to a second end of the eye shaft opposite the first end of the eye shaft; and a rod coupled to a second portion of the adjustment shaft opposite the first portion of the adjustment shaft; inserting a distal end of the rod through the lumen of the collar such that the suture is disposed between and inner surface of the collar and an outer surface of the rod; extending the first and second ends of the suture to the eyelet of the eye shaft; coupling the first and second ends of the suture to each other and to the eyelet of the eye shaft; adjusting the adjustment shaft relative to the eye shaft to lengthen the loading device, thereby tightening the suture and causing the crowns of the first stent ring at the first end of the stent-graft to converge radially inwardly; positioning a distal end of a graft cover defining a graft cover lumen adjacent the eyelet of the eye shaft of the loading device; advancing the loading device with the crowns converged into the distal end of the graft cover until the first end of the stent-graft is received within the graft cover lumen; advancing the loading device and stent-graft into the lumen of the graft cover until the stent-graft is disposed in the graft cover lumen in a radially compressed configuration; severing the suture adj acent the eyelet of the eye shaft; removing the suture from the loading device and the stent-graft; and removing the loading device from within the stent-graft and the graft cover.

In another example hereof, in the method according to any of the preceding or following examples, coupling the suture to the eyelet includes inserting the suture through a gap and into an eye of the eyelet.

In another example hereof, in the method according to any of the preceding or following examples, the second end of the eye shaft includes a first threaded portion and the first portion of the adjustment shaft includes a second threaded portion configured to engage the first threaded portion, wherein adjusting the adjustment shaft relative to the eye shaft to lengthen the loading device includes rotating the adjustment shaft and/or the eye shaft such that the first threaded portion and the second threaded portion unthread from each other, thereby translating the adjustment shaft and the eye shaft away from each other.

In another example hereof, in the method according to any of the preceding or following examples, the eye shaft includes a cavity at the second end, wherein the first threaded portion is disposed within the cavity, and wherein the first portion of the adjustment shaft is received within the cavity such that the second threaded portion engages the first threaded portion to adjustably couple the adjustment shaft and the eye shaft.

In another example hereof, in the method according to any of the preceding or following examples, the first end and the second end of the suture are coupled to each other adjacent the eyelet, and wherein a middle portion of the suture is threaded through the crowns at the first end of the stent-graft.

In another example hereof, in the method according to any of the preceding or following examples, weaving the suture through the crowns includes weaving a plurality of sutures through the crowns.

In another example hereof, the present disclosure relates to a method of loading a stent-graft into a graft cover including: coupling a suture to a first end of a stent-graft in a radially expanded configuration by weaving the suture through adjacent crowns of a first stent ring at the first end of the stent-graft; advancing a loading device through the stent-graft such that a proximal portion of the loading device is proximal of the stent-graft and a distal portion of the stent graft is distal of the stent-graft; advancing a first end and a second end of the suture in a first direction through a first eyelet at a proximal end of a proximal shaft of the loading device; advancing the first and second ends of the suture from the first eyelet in a second direction opposite the first direction along the proximal shaft, past the stent-graft, along a distal adjustment shaft of the loading device, and along a distal eye shaft of the loading device adjustably coupled to the distal adjustment shaft to a second eyelet at a distal end of the distal eye shaft; coupling the first and second ends of the suture to each other and to the second eyelet; lengthening the loading device, thereby tightening the suture and causing the crowns of the first stent ring at the first end of the stent-graft to converge radially inwardly; positioning a distal end of a graft cover defining a graft cover lumen adjacent the first eyelet of the proximal shaft of the loading device; advancing the loading device with the crowns converged into the distal end of the graft cover until the first end of the stent-graft is received within the graft cover lumen; advancing the loading device and stent-graft into the lumen of the graft cover until the stent-graft is disposed in the graft cover lumen in a radially compressed configuration; severing the suture adjacent the eyelet of the distal eye shaft; removing the suture from the loading device and the stent-graft; and removing the loading device from within the stent-graft and the graft cover.

In another example hereof, in the method according to any of the preceding or following examples, the graft cover is part of a delivery device, and wherein advancing the loading device into the graft cover includes advancing the proximal shaft into a stability lumen of a stability shaft of the delivery device disposed within the graft cover, and advancing the stent-graft coupled to the loading device such that the stability shaft is disposed within the stent-graft such that the stent-graft is disposed between an inner surface of the graft cover and an outer surface of the stability shaft.

In another example hereof, in the method according to any of the preceding or following examples, lengthening the loading device includes adjusting the distal adjustment shaft relative to the distal eye shaft to such that the distal adjustment shaft and the distal eye shaft move away from each other to lengthen the loading device.

In another example hereof, in the method according to any of the preceding or following examples, a proximal end of the distal eye shaft includes a first threaded portion and a distal end of the distal adjustment shaft includes a second threaded portion configured to engage the first threaded portion, wherein adjusting the distal adjustment shaft relative to the distal eye shaft to lengthen the loading device includes rotating the distal adjustment shaft and/or the distal eye shaft such that the first threaded portion and the second threaded portion unthread from each other, thereby translating the distal adjustment shaft and the distal eye shaft away from each other.

In another example hereof, in the method according to any of the preceding or following examples, the distal eye shaft includes a cavity at the proximal end thereof, wherein the first threaded portion is disposed within the cavity, and wherein the distal end of the distal adjustment shaft is received within the cavity such that the second threaded portion engages the first threaded portion to adjustably couple the distal adjustment shaft and the distal eye shaft.

In another example hereof, in the method according to any of the preceding or following examples, the proximal shaft includes a proximal adjustment shaft adjustably coupled to a proximal eye shaft, wherein the first eyelet is disposed at a proximal end of the proximal eye shaft, and wherein lengthening the loading device includes adjusting the distal adjustment shaft relative to the distal eye shaft such that the distal adjustment shaft and the distal eye shaft move away from each other to lengthen the loading device and/or adjusting the proximal adjustment shaft relative to the proximal eye shaft such that the proximal adjustment shaft and the proximal eye shaft move away from each other to lengthen the loading device.

In another example hereof, in the method according to any of the preceding or following examples, weaving the suture through the crowns includes weaving a plurality of sutures through the crowns.

### BRIEF DESCRIPTION OF DRAWINGS

FIG. 1 depicts an exploded view of a loading device in accordance with embodiments hereof.
FIG. 2A depicts a longitudinal cross-sectional view of an eye shaft of the loading device of FIG. 1.
FIG. 2B depicts another longitudinal cross-sectional view of the eye shaft of the loading device of FIG. 1.
FIG. 2C depicts a cross-sectional view of the eye shaft taken at line 2C-2C of FIG. 2A.
FIG. 2D depicts a close-up side view of an eyelet of the eye shaft of FIG. 2A.
FIG. 3 depicts a perspective view of an adjustment shaft of the loading device of FIG. 1.
FIG. 4A depicts a side view of the adjustment shaft of FIG. 3.
FIG. 4B depicts a cross-sectional view of the adjustment shaft taken at line 4B-4B of FIG. 4A.
FIG. 4C depicts a cross-sectional view of the adjustment shaft taken at line 4C-4C of FIG. 4A.
FIG. 4D depicts a cross-sectional view of the adjustment shaft taken at line 4D-4D of FIG. 4A.
FIG. 5A depicts a perspective view of a rod of the loading device of FIG. 1.
FIG. 5B depicts a side view of the rod of FIG. 5A.
FIG. 5C depicts a cross-sectional view of the rod taken at line 5C-5C of FIG. 5B.
FIG. 6A depicts a perspective view of a collar of the loading device of FIG. 1.
FIG. 6B depicts a longitudinal cross-sectional view of the collar of FIG. 6A.
FIG. 6C depicts a cross-sectional view of the collar taken at line 6C-6C of FIG. 6B.
FIG. 7A depicts an exploded view of the loading device of FIG. 1.
FIG. 7B depicts a perspective view of the loading device of FIG. 7A in an assembled configuration.
FIG. 8 depicts an exploded view of a loading device according to embodiments hereof.
FIG. 9 depicts an exploded view of a loading device according to embodiments hereof.
FIG. 10A depicts a side view of an exemplary stent-graft in a radially expanded configuration.
FIG. 10B depicts a side view of the exemplary stent-graft of FIG. 9A in a radially compressed configuration.
FIG. 10C depicts a side view of the exemplary stent-graft of FIG. 9A in a loading configuration.
FIG. 11 is a flow chart showing a method of loading a stent-graft into a graft cover according to embodiments hereof.
FIG. 12A depicts a side view of the exemplary stent-graft of FIG. 10A with a suture coupled thereto.
FIG. 12B depicts an end view of the exemplary stent-graft of FIG 10A with a plurality of sutures coupled thereto.
FIGS. 13A-17D depict several illustrations of the method of FIG. 11 for loading a stent-graft into a graft cover according to embodiments hereof.
FIG. 18 is a flow chart showing a method of loading a stent-graft into a graft cover of a delivery device according to embodiments hereof.
FIGS. 19A-19K depict several illustrations of the method of FIG. 18 for loading a stent-graft into a graft cover of a delivery device according to embodiments hereof.

### DETAILED DESCRIPTION

Specific embodiments of the present invention are now described with reference to the figures, wherein like reference numbers indicate identical or functionally similar elements. The terms "distal" and "proximal", when used in the following description to refer to a loading device, are with respect to a position or direction relative to a delivery device for which the loading device is used. Thus, "distal" and "distally" refer to positions distant from, or in a direction away from, a handle of the delivery device, and "proximal" and "proximally" refer to positions near, or in a direction toward, the handle of the delivery device.

The following detailed description is merely exemplary in nature and is not intended to limit the invention or the application and uses of the invention. Furthermore, there is no intention to be bound by any expressed or implied theory presented in the preceding Technical Field, Background, Summary, or the following Detailed Description.

As referred to herein, an exemplary stent-graft used in accordance with and/or as part of the various devices, devices, methods and embodiments of the present disclosure may include a wide variety of different configurations, such as an aortic stent-graft having single or multiple lumens. The stent-graft described herein is not meant to limit the usefulness of the described devices for use with other stent-graft configurations.

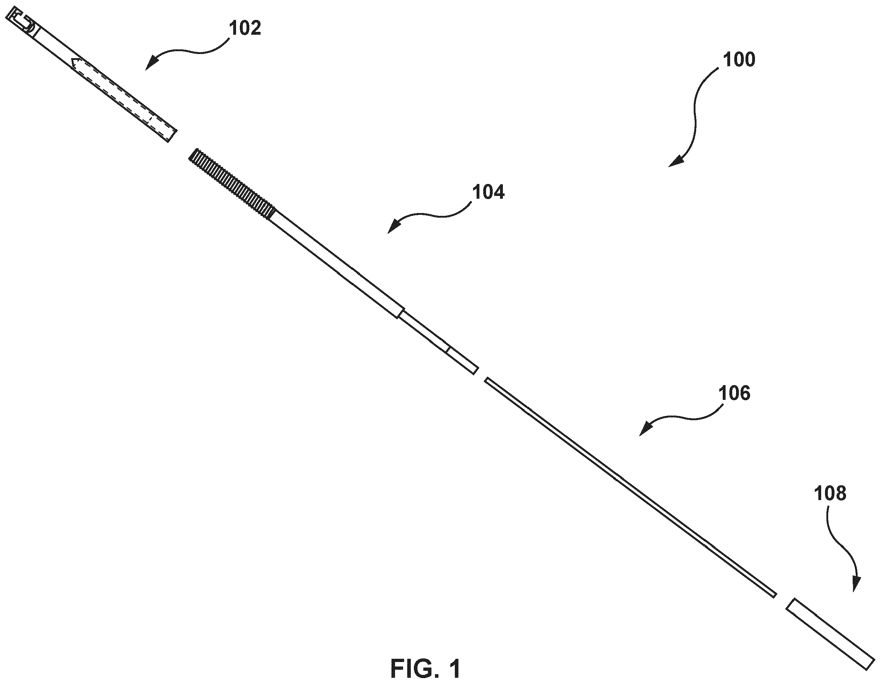

FIGS. 1-6C illustrate a loading device 100 for loading a stent-graft into a graft cover, according to embodiments hereof. One skilled in the art will realize that FIGS. 1A-6C illustrate one example of a loading device, that the devices and methods disclosed herein can be used with other loading devices, and that existing components illustrated in FIGS. 1A-6C may be removed and/or additional components may be added to the loading device 100. The loading device 100 includes an eye shaft 102, an adjustment shaft 104, a rod 106, and a collar 108 as illustrated in FIG. 1.

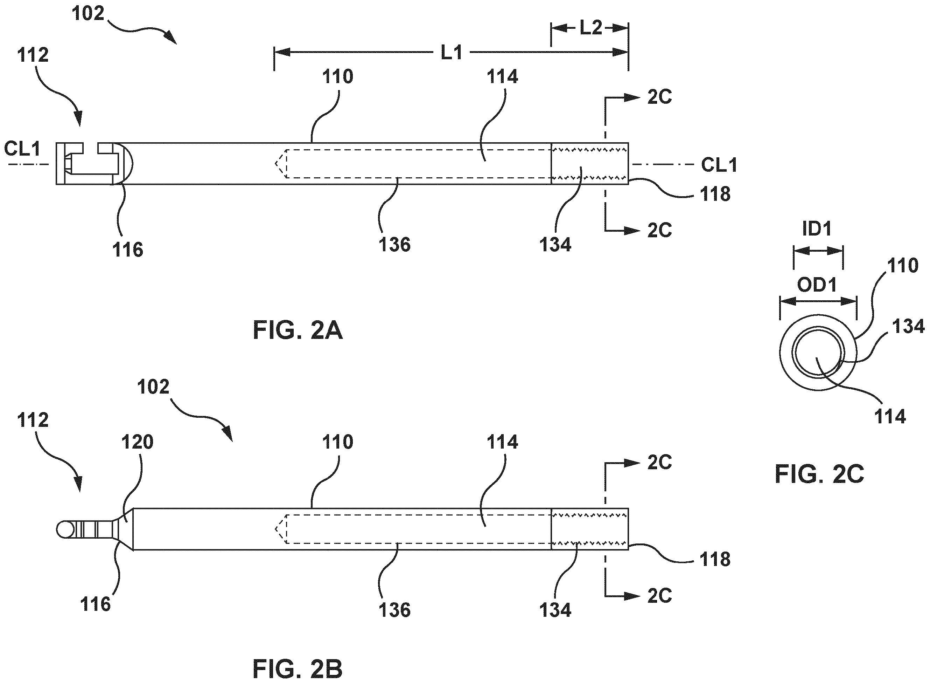

Referring to FIGS. 2A-2C, the eye shaft 102 includes a body 110, an eyelet 112, and a cavity 114. The body 110 is substantially cylindrical and extends from a proximal end 116 to a distal end 118. The eyelet 112 is disposed and extends proximally from the proximal end 116 of the body 110. The cavity 114 extends from the distal end 118 of the body 110 proximally through the body 110, along a longitudinal centerline CL1 of eye shaft 102. The cavity 114 extends proximally for a first length L1, and has an inner diameter ID1, as best seen in FIG. 2C, wherein inner diameter ID1 is smaller than an outer diameter OD1 of the body 110. The cavity 114 is configured to receive a proximal section of the adjustment shaft 104 therein, as described in greater detail below. The distal end 118 of the body 110 includes a first thread 134 along an inner surface of the wall thereof, *i.e.*, facing the cavity 114. The first thread 134 is disposed within the cavity 114 from the distal end 118 of the body proximally for a second length L2 of cavity 114, wherein the second length L2 is shorter than the first length L1. The first thread 134 extends radially inward within the cavity 114 and is configured to engage a second thread 148 of the adjustment shaft 104 as described below. The first thread 134 may be formed for example, and not by way of limitation, as an integral section of the cavity 114 of the eye shaft 102, or as a separate unit coupled to the cavity 114 for example, and not by way of limitation, by adhesives, welding, or other methods as appropriate. While the first thread 134 is shown in FIGS. 2A and 2B with a specific gender, hardness, thread form, thread angle, lead, pitch, and start, this is not meant to limit the design and other configurations and combinations may be utilized. A tapered portion 120 at the proximal end 116 of body 110 transitions the body 110 to the eyelet 112. In embodiments, the first length L1 may be about 1.35 inches, the second length L2 may be about 0.280 inch, the outer diameter OD1 may be about 0.156 inch, and inner diameter ID1 may be about 0.116 inch. The inner diameter ID1 refers to the inner diameter of the cavity 114 without the first thread 134. As noted above, the first thread 134 may be an insert, for example, and not by way of limitation, a heli-coil insert of size 4-40 (nominal 0.12-inch diameter and 3-inch length). However, this is not meant to be limiting, and other sizes may be used provided other parts of the loading device 100 are matched (e.g., the outer diameter of the threaded portion of the adjustment shaft must fit within the cavity 114).

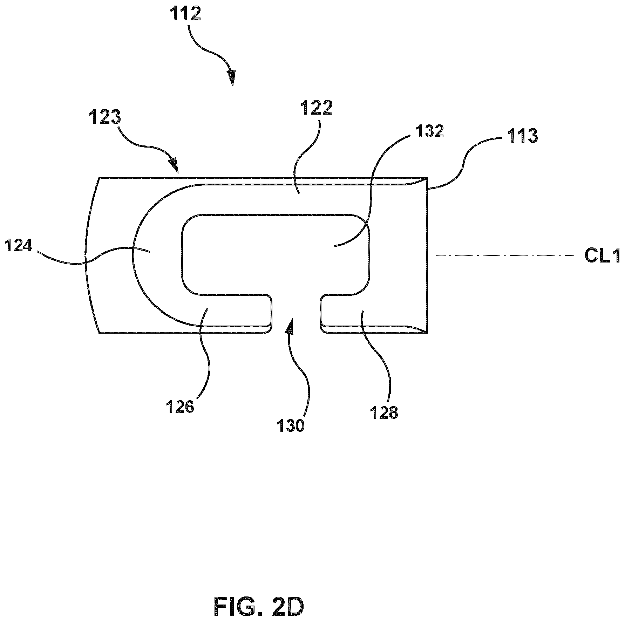

The eyelet 112 of the eye shaft 102 is illustrated in greater detail in FIG. 2D. The eyelet 112 includes a base 113, a first leg 122, a second leg 124, a third leg 126, and a fourth leg 128. The base 113 extends proximally from the proximal end 116 of the body 110 and is substantially perpendicular to the longitudinal centerline CL1 of the eye shaft 102. The first leg 122 of the eyelet 102 extends proximally from the base 113, is substantially parallel to longitudinal centerline CL1 of eye shaft 102 and is spaced to one side of longitudinal centerline CL1. The second leg 124 extends substantially perpendicular to the first leg 122 at a proximal section 123 of the first leg 122 and extends across longitudinal centerline CL1 of the eye shaft 102 for a distance substantially equal to the outer diameter OD1 of the body 110. The third leg 126 extends distally from the second leg 124, substantially parallel to the longitudinal centerline CL1 of the eye shaft 102, and is spaced to one side of longitudinal centerline CL1 of the eye shaft 102 opposite the first leg 122. The fourth leg 128 extends proximally from the base 113, extending substantially parallel to the longitudinal centerline CL1 of the eye shaft 102, but spaced to one side of longitudinal centerline CL1 such that fourth leg 128 and third leg 126 are longitudinally aligned. Third leg 126 extends distally and fourth leg 128 extends proximally such that an opening or gap 130 is formed, providing unrestricted access to an eye 132 of the eyelet 112 such that the eyelet 112 is an open eyelet. The eye 132 is defined by the base 113, the first leg 122, the second leg 124, the third leg 126, the fourth leg 128, and the gap 130. The gap 130 and the eye 132 are configured to receive sutures therethrough. The eyelet 112 may be formed for example, and not by way of limitation, as an integral section of the eye shaft 102, or as a separate unit coupled to the proximal end 116 of the body 110 for example, and not by way of limitation, by adhesives, welding, or other methods as appropriate. While the eyelet 112 is described herein such that the gap 130 is formed by the third leg 126 and the fourth leg 128, this is not meant to be limiting, and in other embodiments the third leg 126 may extend distally further such that the gap 130 is formed between the third leg 126 and the base 113, or the gap 130 may be eliminated such that the eyelet 112 is a closed eyelet 112. In such an embodiment, eye 132 is defined by the base 113, the first leg 122, the second leg 124, and the third leg 126 (or the fourth leg 128).

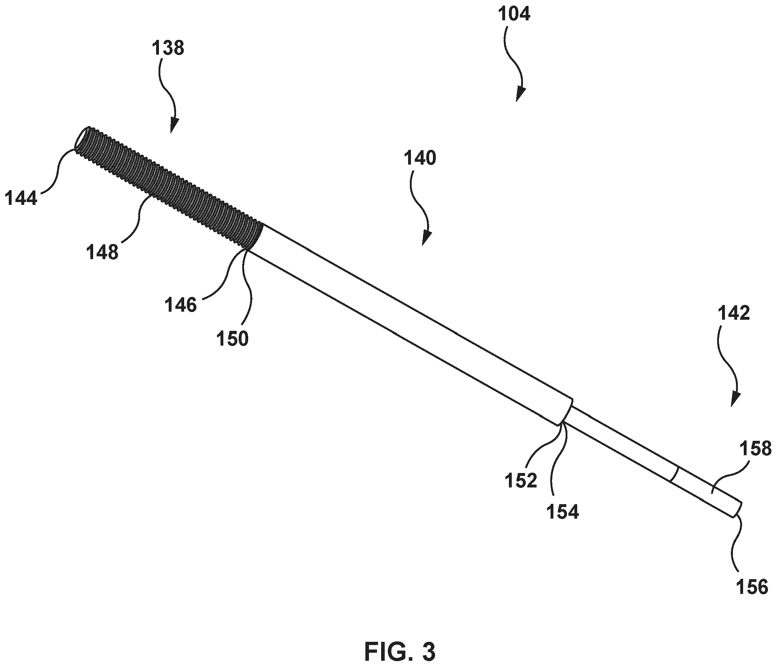

The adjustment shaft 104 is shown in detail in FIGS. 3-4D. The adjustment shaft 104 includes a threaded section 138, a center section 140, and a rod section 142. The adjustment shaft 104 may be formed as an integral unit or may be formed from separate pieces that are coupled to each other by, for example, and not by way of limitation, by adhesives, welding, or other methods as appropriate.

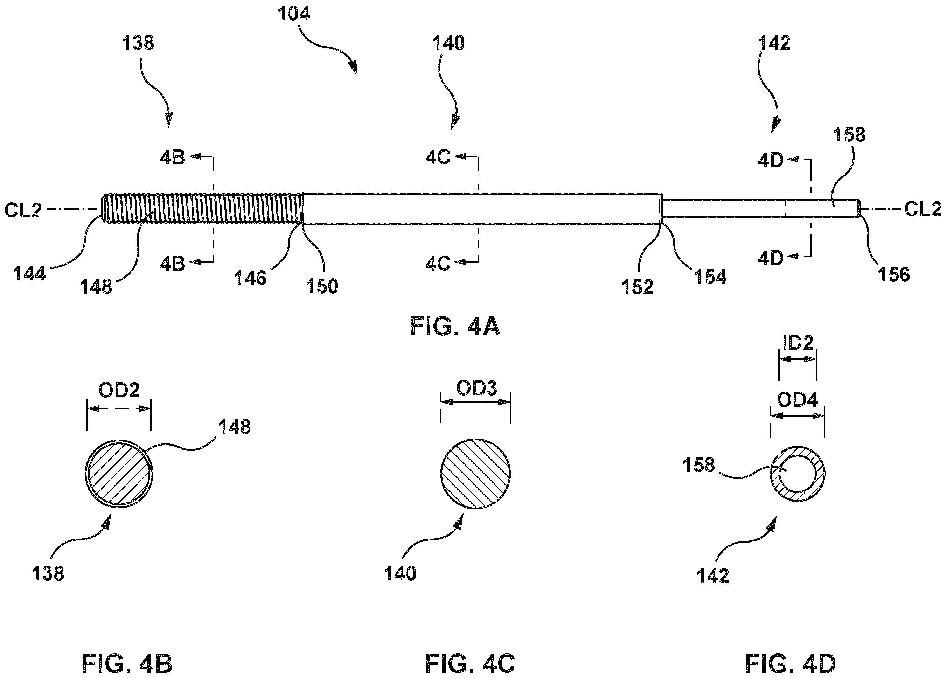

The threaded section 138 includes a proximal end 144 and a distal end 146. A second thread 148 is disposed on an outer surface of the threaded section 138 and extends longitudinally from the proximal end 144 to the distal end 146. The threaded section 138 has an outer diameter of OD2, as shown in FIG. 4B, wherein OD2 is smaller than the inner diameter ID1 of the cavity 114. The outer diameter OD2 of the second thread 148 is sized and shaped such that the threaded section 138 may threadably engage the threaded portion of the cavity 114. The second thread 148 may be any suitable design corresponding to the first thread 134 of the eye shaft 102. The second thread 148 is configured to engage the first thread 134 of eye shaft 102 to adjustably couple the adjustment shaft 104 to the eye shaft 102. The second thread 148 may be formed for example, and not by way of limitation, as an integral section of the threaded section 138 of the adjustment shaft 104, or as a separate unit coupled to threaded section 138 for example, and not by way of limitation, by adhesives, welding, or other methods as appropriate. While the second thread 148 is shown in FIGS. 3 and 4A with a specific gender, hardness, thread form, thread angle, lead, pitch, and start, this is not meant to limit the design and other configurations and combinations may be utilized.

The center section 140 of the adjustment shaft 104 includes a proximal end 150 and a distal end 152. The proximal end 150 is coupled to the distal end 146 of the threaded section 138. The center section 140 may be a cylindrical rod with an outer diameter of OD3, and shown in FIG. 4C. The outer diameter OD3 of the center section 140 is larger than outer diameter OD2 of the threaded section 138, and may be substantially equivalent to the outer diameter OD1 of the eye shaft 102 such that when the adjustment shaft 104 and the eye shaft 102 are coupled, full insertion of the threaded section 138 into the cavity 114 results in the proximal end 150 of the center section 140 abutting the distal end 118 of the body eye shaft 102. In an embodiment, the outer diameter OD3 of the center section 140 is about 0.125 inch. The adjustment shaft 104 and the eye shaft 102 are configured to provide an interface for user manipulation such that rotating the adjustment shaft 104 and/or the eye shaft 102 relative each other adjusts the overall length of the assembled loading device 100, as described below.

With reference to FIGS. 4A and 4D, the rod section 142 of the adjustment shaft 104 includes a proximal end 154 coupled to the distal end 152 of the center section 140, and a distal end 156 opposite the proximal end 154. The rod section 142 further includes a rod cavity 158 extending proximally from the distal end 156, and along a centerline CL2 of adjustment shaft 104. The rod section 142 has an outer diameter OD4. In embodiments, the outer diameter OD4 of the rod section 142 is smaller than the outer diameter OD3 of the center section 140. The rod section 142 at the rod cavity 158 has an inner diameter ID2 (i.e., diameter of the rod cavity 158) that is smaller than the outer diameter OD4 of the rod section 142. The rod cavity 158 is configured (e.g., sized and shaped) to receive a proximal section of the rod 106 therein to couple the adjustment shaft 104 to the rod 106, as described below. In a non-limiting example, the outer diameter OD3 of the rod section 142 is about 0.068 inch and the inner diameter ID3 of the rod section 142 at the rod cavity 158 (i.e., diameter of the rod cavity 158) is about 0.0469 inch. Those skilled in the art will recognize that the sizes provided are merely examples, that other sizes may be utilized, and that the relative sizes of some of the parts are desirable as described.

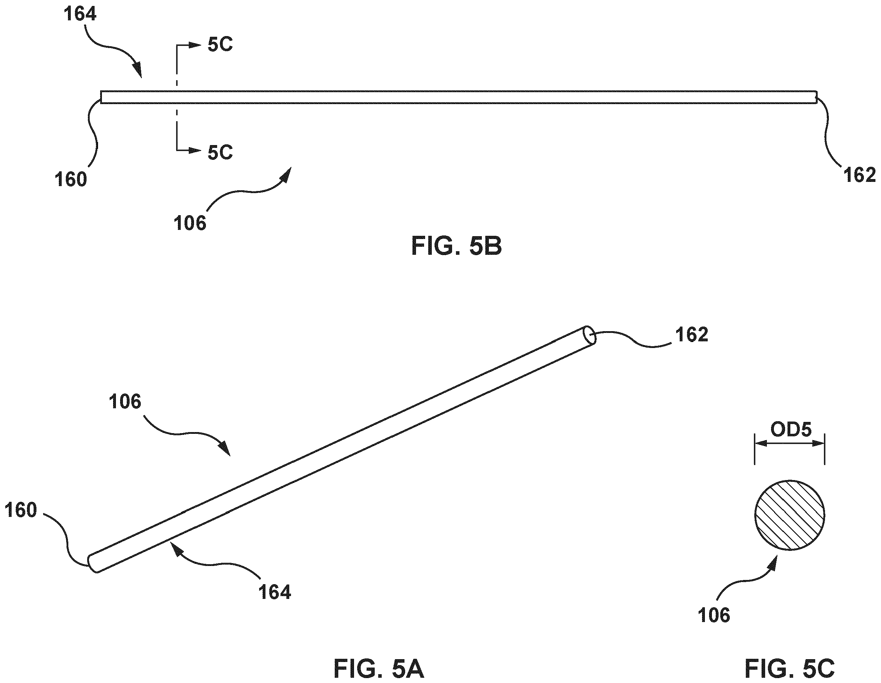

The rod 106 is illustrated in FIGS. 5A and 5B, and includes a proximal end 160 and a distal end 162. The rod 106 is substantially cylindrical, having an outer diameter OD5 that is approximately equal to or smaller than the inner diameter ID2 of the rod cavity 158. A proximal section 164 of the rod 106 is sized and shaped to be disposed within the rod cavity 158 of the adjustment shaft 104 such that the rod 106 is coupled to the adjustment shaft 104. In an example, the outer diameter OD5 of the rod shaft is about 0.0469 inch (i.e., approximately equal to the diameter of the rod cavity 158). In an embodiment, the rod 106 is configured to be coupled to the adjustment shaft 104 within the rod cavity 158 via friction, but other coupling methods are possible. For example, the rod 106 may be coupled to the adjustment shaft 104 within the rod cavity 158 via adhesives, welding, or other coupling methods as appropriate.

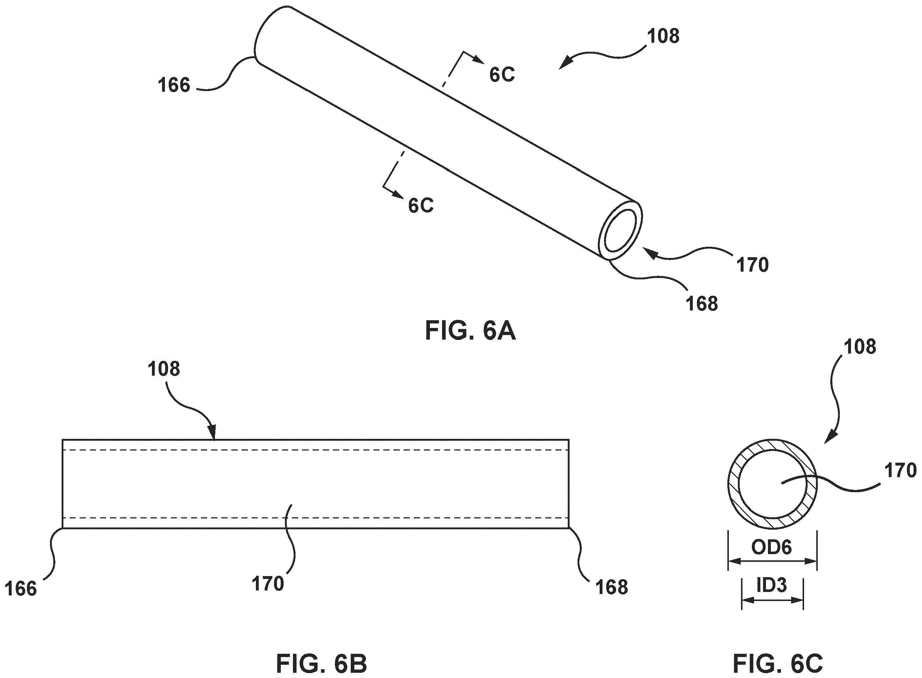

FIGS. 6A-6C illustrate the collar 108 of the loading device 100. The collar 108 includes a proximal end 166, a distal end 168, and a lumen 170 disposed therethrough from the proximal end 166 to the distal end 168. Thus, in the embodiment shown, the collar is a hollow, cylindrical tube. The collar 108 has an outer diameter of OD6 and an inner diameter ID3 defining the lumen 170. The inner diameter ID3 of the collar 108 is larger than the outer diameter OD5 of the rod 106 and larger than the outer diameter OD4 of the rod section 142 of the adjustment shaft 104. The inner diameter ID3 of collar 108 is also smaller than the outer diameter OD3 of the center section 140 of the adjustment shaft 104. In a non-limiting example, the outer diameter OD6 of the collar 108 is about 0.13 inch and the inner diameter ID3 of the collar 108 is about 0.10 inch. When the loading device 100 is assembled, the collar 108 is configured to be disposed over the rod 106 and the rod section 142 of the adjustment shaft 104, with the proximal end 166 of the collar 108 abutting the distal end 152 of the center section 140 of the adjustment shaft 104. Moreover, when the loading device 100 is assembled, inner diameter ID3 of the collar 108 (i.e., the diameter of the lumen 170) is sufficiently larger than the outer diameter OD4 of the rod section 142 of the adjustment shaft 104 such that a suture (described below) fits within the lumen 170 of the collar 108 with the collar 108 disposed over the rod section 142 (i.e., between the inner surface of the collar 108 and the outer surface of the rod section 142).

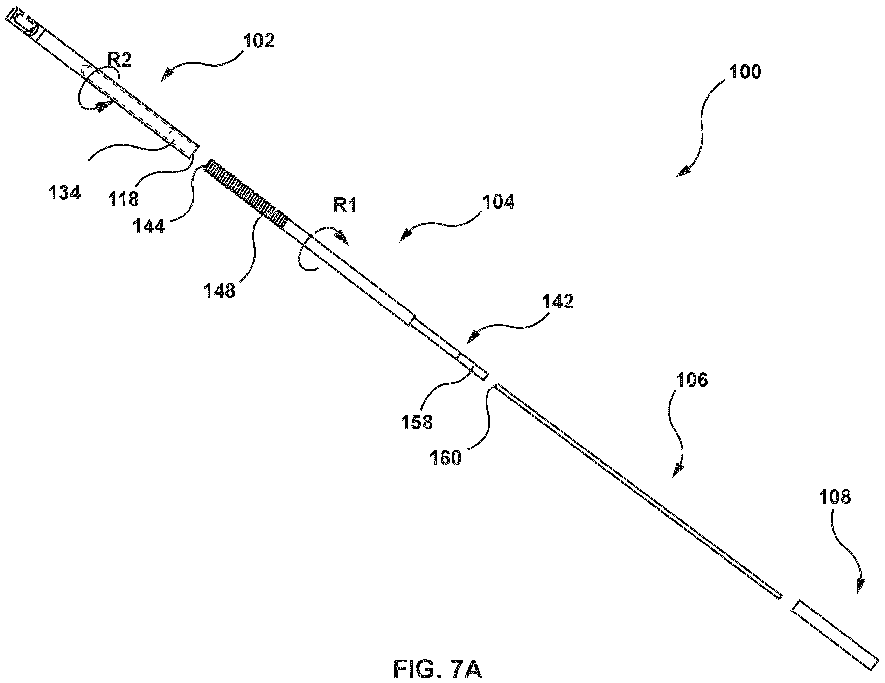

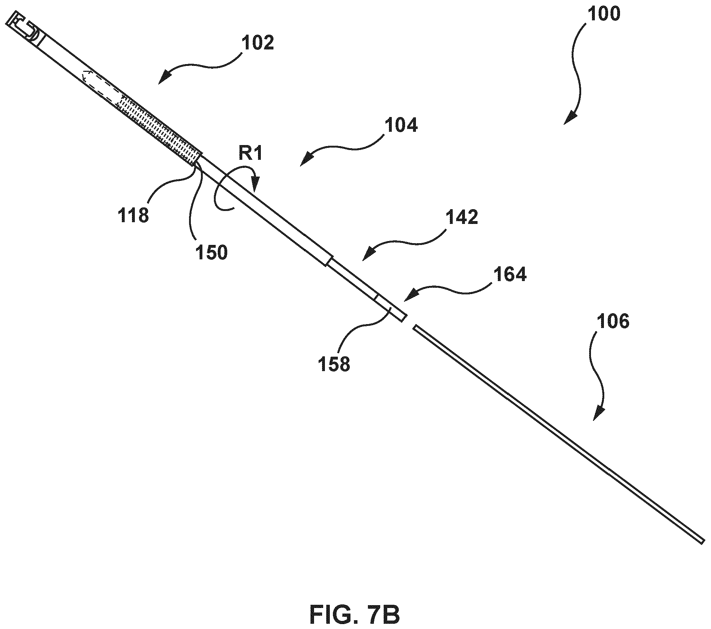

With reference back to FIG. 1 and to FIGS. 7A-7B, assembly of the loading device 100 will now be described in no particular order. The proximal end 144 of the adjustment shaft 104 is disposed at the distal end 118 of eye shaft 102. As depicted in FIG. 7A, the adjustment shaft 104 and/or the eye shaft 102 are rotated in opposite directions (e.g. a first direction R1 and a second direction R2) such that the second thread 148 of the adjustment shaft 104 engages the first thread 134 of the eye shaft 102 to adjustably couple the adjustment shaft 104 to the eye shaft 102. The adjustment shaft 104 and/or the eye shaft 102 may be rotated relative to each other until the proximal end 150 of the center section 140 of the adjustment shaft 104 abuts the distal end 118 of the eye shaft 102, as shown in FIG. 7B, although it is not necessary.

In another step, the rod 106 is coupled to the adjustment shaft 104. The proximal end 160 of the rod 106 inserted into the rod cavity 158 of the rod section 142 of the adjustment shaft 104. In an embodiment, due to the relative sizes of the rod 106 and the rod cavity 158, the rod 106 is frictionally coupled to the rod section 142 (i.e., force fit). However, as noted above, in other embodiments, the rod 106 and the adjustment shaft 104 may be coupled in other ways. It is noted that the assembly of the loading device 100 as described above may be completed during manufacturing and thus need not be done during loading of the stent-graft 300 into the graft cover, which is described below. However, this is not meant to be limiting and the assembly of the loading device 100 may be done at the loading site.

Thus, with the loading device 100 assembled and configured to be used to load a stent-graft into a graft cover, the eye shaft 102 is disposed at a proximal end of the loading device 100. The adjustment shaft 104 is adjustably coupled to the eye shaft 102. More specifically, in the assembled state of the loading device 100, the threaded section 138 of the adjustment shaft 104 is disposed within the cavity 114 of the body 110 of the eye shaft 102, with the second thread 148 of the adjustment shaft 104 engaged with the first thread 134 of the eye shaft 102 to adjustably couple the adjustment shaft 104 to the eye shaft 102. Further, in the assembled state, the rod 106 is coupled to the distal end 156 of the adjustment shaft 104 with a proximal section of the rod 106 disposed within the rod cavity 158 of the rod section 142 of the adjustment shaft 104. The collar 108 is disposed over the rod 106 and the rod section 142 of the adjustment shaft 104 such that the proximal end 166 of the collar 108 is adjacent the distal end 152 of the center section 140 of the adjustment shaft 104. With the loading device 100 assembled, rotational movement of the adjustment shaft 104 and/or the eye shaft 102 relative to each other causes the second thread 148 to advance or retract within the cavity 114 of the eye shaft 102 such that the overall length of the loading device 100 may be adjusted by a user, as described in greater detail below.

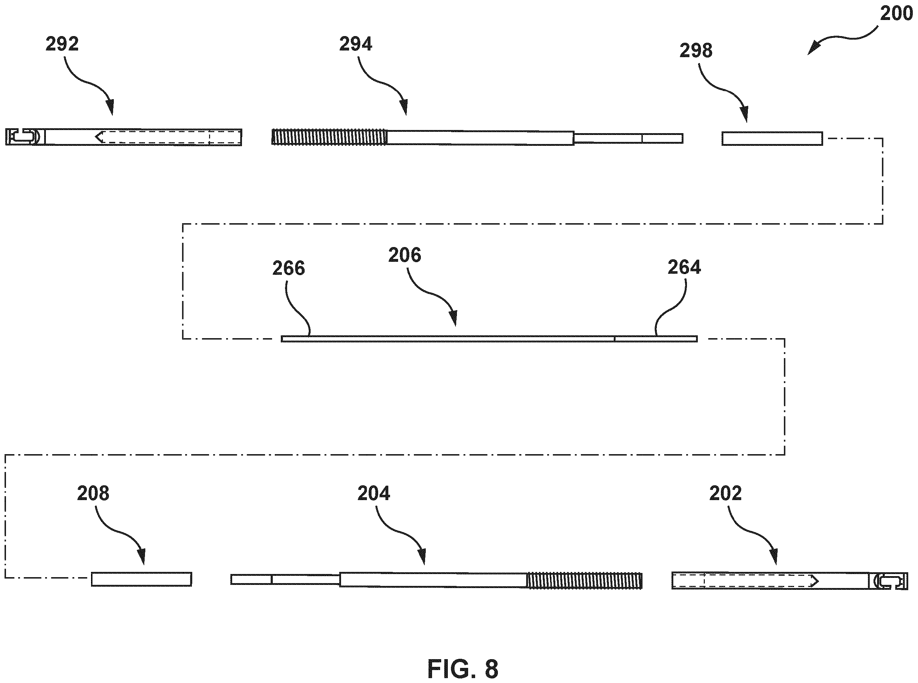

FIG. 8 illustrates a loading device 200 for loading a stent-graft into a graft cover, according to another embodiment hereof. The loading device 200 includes a proximal eye shaft 202 coupled to a proximal end of a proximal adjustment shaft 204, coupled to a proximal portion 264 of a rod 206. A distal portion 266 of the rod 206 is coupled to a proximal end of a distal adjustment shaft 294, which is coupled to a proximal end of a distal eye shaft 292. A proximal collar 208 is disposed over a distal portion of the proximal adjustment shaft 204 (*i.e.,* over the rod section of the proximal adjustment shaft 204). A distal collar 298 is disposed over a proximal portion of the distal adjustment shaft 294 (*i.e.,* over the rod section of the distal adjustment shaft 294). Although the loading device 200 is described as including the distal collar 298, the distal collar 298 is not needed. In particular, the collar on the end of the stent-graft that the suture is coupled to, in this case the proximal collar 208, is useful to prevent the stent-graft from moving proximally, as described below. However, both collars may be provided with the loading device 200 for convenience (*e*.*g*., either end of the loading device 200 can be used as the proximal end or the distal end). One skilled in the art will realize that FIG. 8 illustrates one example of a loading device, that the devices and methods disclosed herein can be used with other loading devices, and that existing components illustrated in FIG. 8 may be removed and/or additional components may be added to the loading device 200.

The elements of the loading device 200 as shown in FIG. 8 are the same as the corresponding elements of the loading device 100 described above. The difference in the loading device 200 and is that there are two of each part, except for the rod 206, of which there is one, with the proximal elements coupled the proximal portion 264 of the rod 206 and the distal elements coupled to the distal portion 266 of the rod 206. Thus, the details of each element (*i.e.*, the details of the proximal and distal eye shafts 202, 292, the proximal and distal adjustment shafts 204, 294, the proximal and distal collars 208, 298, and the rod 206) are the same as the details described above with respect to FIGS. 1-7B. Therefore, they will not be repeated here and are incorporated in their entirety into the embodiment of FIG. 8.

With reference back to FIG. 8, the assembled loading device 200 will now be described. When the loading device 200 is assembled and configured to load a stent-graft into a delivery device, the proximal eye shaft 202 is disposed at a proximal end of loading device 200 and the distal eye shaft 292 is disposed at a distal end of loading device 200. The proximal eye shaft 202 is adjustably coupled to a proximal end of the proximal adjustment shaft 204. More specifically, a thread section of proximal adjustment shaft 204 is disposed within a cavity of a body 210 of the proximal eye shaft 202, with a second thread of the proximal adjustment shaft engaged with the first thread of proximal eye shaft 202 such that proximal eye shaft 202 is adjustably coupled thereto, as described above with respect to FIGS. 1-6C. A proximal portion 264 of the rod 206 is disposed within a rod cavity of a rod section of the proximal adjustment shaft 204, as also described above with respect to FIGS. 1-6C. The proximal collar 208 is disposed over the rod 206 and the rod section of the proximal adjustment shaft 204 such that a proximal end of the proximal collar 208 is disposed adjacent a distal end of a center section of the proximal adjustment shaft 204, as also described above with respect to FIGS. 1-6C. The distal adjustment shaft 294 is coupled to the distal portion 266 of the rod 206, with the distal portion 266 of the rod 206 disposed within a rod cavity at a proximal end of a rod section of the distal adjustment shaft 294, as described above with respect to FIGS. 1-6C. The distal eye shaft 292 is adjustably coupled to a distal end of the distal adjustment shaft 294. More specifically, a threaded section of the distal adjustment shaft 294 is disposed within a cavity of the distal eye shaft 292, with a thread of the distal adjustment shaft 294 engaging a corresponding thread within the cavity of the distal eye shaft 292, as described above with respect to FIGS. 1-6C.

With the loading device 200 assembled, rotational movement of proximal and/or distal adjustment shafts 204, 294, causes the corresponding threads of the proximal and/or distal adjustment shafts 204, 294 to advance or retract within the respective cavities of the proximal eye shaft 202 and distal eye shaft 292 such that an overall length of the loading device 200 may be adjusted by a user.

Those skilled in the art will recognize that the proximal and distal elements of the loading device 200 shown in FIG. 8 need not be the same. In particular, as explained in more detail below, the threaded shafts (i.e., the eye shaft and the adjustment shaft) are only needed on one side of the loading device 200, preferably the distal side. Therefore, the proximal side of the loading device 200 need not have threaded, adjustable shafts.

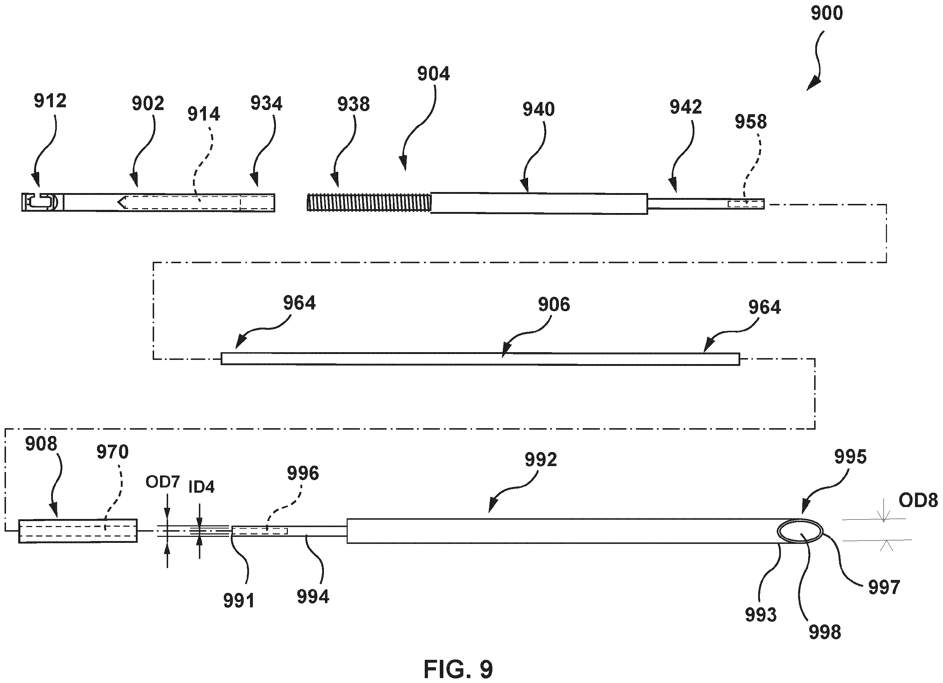

FIG. 9 illustrates a loading device 900 for loading a stent-graft into a graft cover, according to another embodiment hereof, showing an embodiment wherein the proximal elements differ from the distal elements. In particular, the loading device 900 includes a proximal shaft 992 coupled to a proximal portion 964 of a rod 906. A proximal collar 908 is disposed over a proximal portion of the proximal shaft 992 (*i.e.,* over a rod section 994 of the proximal shaft 992). A distal section 966 of the rod 906 is coupled to a proximal end of a distal adjustment shaft 904, which is coupled to a proximal end of a distal eye shaft 902. One skilled in the art will realize that FIG. 9 illustrates one example of a loading device, that the devices and methods disclosed herein can be used with other loading devices, and that existing components illustrated in FIG. 9 may be removed and/or additional components may be added to the loading device 900.

The distal elements of the loading device 900 as shown in FIG. 9 are the same as the corresponding elements of the loading device 100 described above. Accordingly, the distal eye shaft 902 is the same as the eye shaft 102 described above, including a distal eyelet 912 and a cavity 914 including a first thread 934. Thus, the details of the distal eye shaft 902 will not be repeated here, and the details described above with respect to the eye shaft 102 are incorporated herein with respect to the distal eye shaft 902. Similarly, the distal adjustment shaft 904 is the same as the adjustment shaft 104 described above, including a threaded section 938 configured to be coupled to the first thread 934 within the cavity 914 of the distal eye shaft 902, a center section 940, and a rod section 942 including a rod cavity 958. Thus, the details of the distal adjustment shaft 904 will not be repeated here, and the details described above with respect to the adjustment shaft 104 are incorporated herein with respect to the distal adjustment shaft 904.

As briefly discussed above, only one side of the loading device 900 needs to be adjustable. Thus, in the loading device 900, the proximal portion thereof (the proximal shaft 992) is not adjustable. However, it is noted that the overall length of the loading device 900, including the proximal shaft 992, is adjustable via the distal eye shaft 902 and the distal adjustment shaft 904. The proximal shaft 992 of the loading device 900 includes a proximal end 993 and a distal end 991. A proximal eyelet 995 is disposed at the proximal end 993 of the proximal shaft 992 and a cavity 996 is disposed at a rod section 994 at the distal end 991 of the proximal shaft 992, similar to the rod section 142 described above with respect to the adjustment shaft 104 of the loading device 100. Further, in the embodiment of FIG. 9, although it is preferable that the adjustable portion of the loading device 900 is the distal portion, this is not meant to be limiting, and either the proximal portion or the distal portion may be adjustable.

The proximal eyelet 995 in the embodiment shown is defined by a ring 997 having an opening 998 disposed therethrough, as shown in FIG. 9. The proximal eyelet 995 shown is a closed eyelet. However, this is not meant to be limiting, and the proximal eyelet 995 may be an open eyelet similar to the distal eyelet 912. Further, although the proximal eyelet 995 is shown as generally oblong in shape, this is not meant to be limiting, and the proximal eyelet 995 may be other shapes, such as but not limited to, rectangular and circular. As explained in more detail below, the proximal eyelet is configured to receive a suture for radially cinching an end of a stent-graft for loading into a graft cover of a delivery device.

The cavity 996 is disposed at the rod section 994 at the distal end 991 of the proximal shaft 992. Similar to the rod section 142 described above, the rod section 994 of the proximal shaft 992 may have an outer diameter OD7 that is smaller than and outer diameter OD8 of the proximal shaft 992 proximal of the rod section 994. The rod section 994 may have an inner diameter ID4 defining the cavity 996 that is smaller than the outer diameter OD7. The outer diameter OD7 is larger than the outer diameter of the rod 906 (defined as outer diameter OD5 above). In an embodiment, the inner diameter ID4 of the rod section 994 of the proximal shaft 992 defining the cavity 996 may be approximately equal to the outer diameter OD5 of the rod 906 such that the rod 906 may be press fit into the cavity 996 to couple the proximal portion 964 of the rod 906 to the proximal shaft 992. In other embodiments, the inner diameter ID7 of the rod section 994 of the proximal shaft 992 defining the cavity 996 may be slightly larger than the outer diameter OD5 of the rod 906 such that the rod 906 may be inserted into the cavity 996 and coupled thereto, such as by adhesives. In a non-limiting example, the inner diameter ID4 of the proximal shaft 992 defining the cavity 996 may be about 0.0469 inch, matching the outer diameter OD5 of the rod 906.

The proximal collar 908 may be the same as the collar 108 described above, including a lumen 970 extending therethrough. Thus, the details of the proximal collar 908 will not be repeated here, and the details described above with respect to the collar 108 are incorporated herein with respect to the proximal collar 908. As described above, the lumen 970 is sized to fit over the rod section 942 of the proximal shaft 992 such that a suture, as described below, extends between an outer surface of the rod section 942 and an inner surface of the collar. The collar 908 is also sized such that it will abut the proximal shaft 992 where the proximal shaft 992 transitions to the rod section 942 (*i.e.,* the lumen 970 is smaller than the outer diameter OD8).

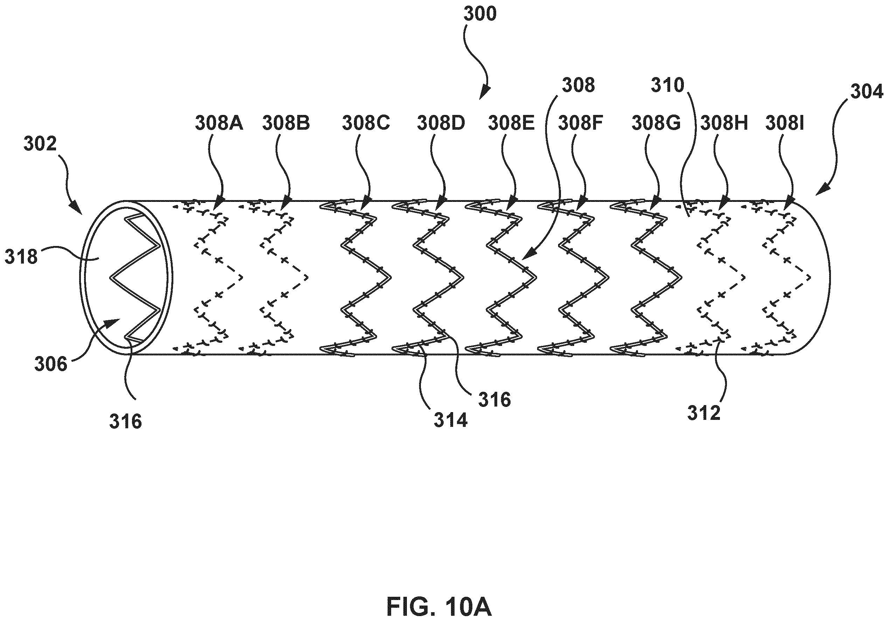

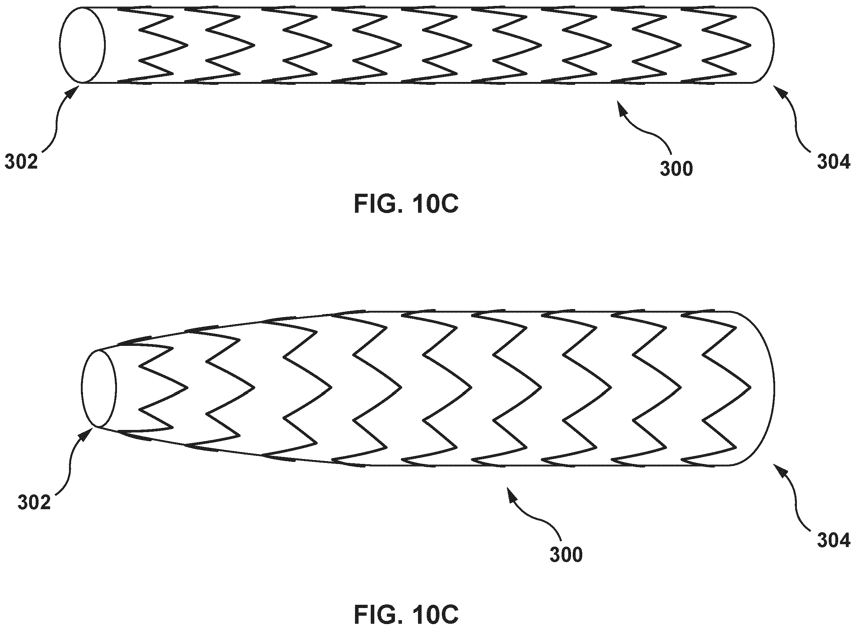

FIGS. 10A-10C show an example stent-graft 300 suitable for use with the loading devices 100, 200, 900. The stent-graft 300 is formed of a graft material 310 and a stent 308 (or plurality of stents 308) coupled to the graft material 310. The stent-graft 300 includes a first end 302, a second end 304, and a lumen 306 extending from the first end 302 to the second end 304, with the lumen 306 being defined by an inner surface 318 of the graft material 310. In the embodiment shown, the stent 308 includes a plurality of stent rings 308A-308H, with each stent ring 308A-308H includes a plurality of struts 314 joined together at bends or crowns 316 to form a zig-zag pattern around a circumference of the graft material 310. The stent 308 is coupled to the graft material 310 via sutures 312, but other coupling devices may be used, such as, but not limited to, adhesives. In other embodiments, the stent 308 may be a single helically wrapped stent that includes a plurality of wraps, and also includes struts and crowns in a zi-zag pattern. Other patterns and arrangements are also contemplated. In the embodiment shown, the stent rings 308A, 308B, 308G, 308H adjacent the first and second ends 302, 304 of the stent-graft 300 are coupled to the inner surface 318 of the graft material 310, and intermediate stent rings 308C-308F are coupled to an outer surface of the graft material 310. However, this is not meant to be limiting, and more or fewer stent rings 308A-308H may be coupled to the outer surface or to the inner surface of the graft material 310. In embodiments, all of the stent rings 308A-308H may be coupled to the inner surface of the graft material 310, or all of the stent rings 308A-308H may be coupled to the outer surface of the graft material 310. Further, there may be more or fewer stent rings. The stent-graft may also include multiple legs at an end of the stent-graft, as known to those skilled in the art.

In embodiments hereof, each of the stent rings 308A-308H is self-expanding to return to a radially expanded state from a radially compressed state. The stent rings 308A-308H may be formed of various materials including, but not limited to stainless steel, nickel-titanium alloys, or other suitable materials. "Self-expanding" as used herein means that a structure has a mechanical memory to return to the radially expanded state.

The graft material 310 is of a generally tubular shape. The graft material 310 may be formed of any suitable graft material, for example, and not limited to, a low porosity woven or knit polyester, DACRON material, expanded polytetrafluoroethylene, polyurethane, silicone, or other suitable materials.

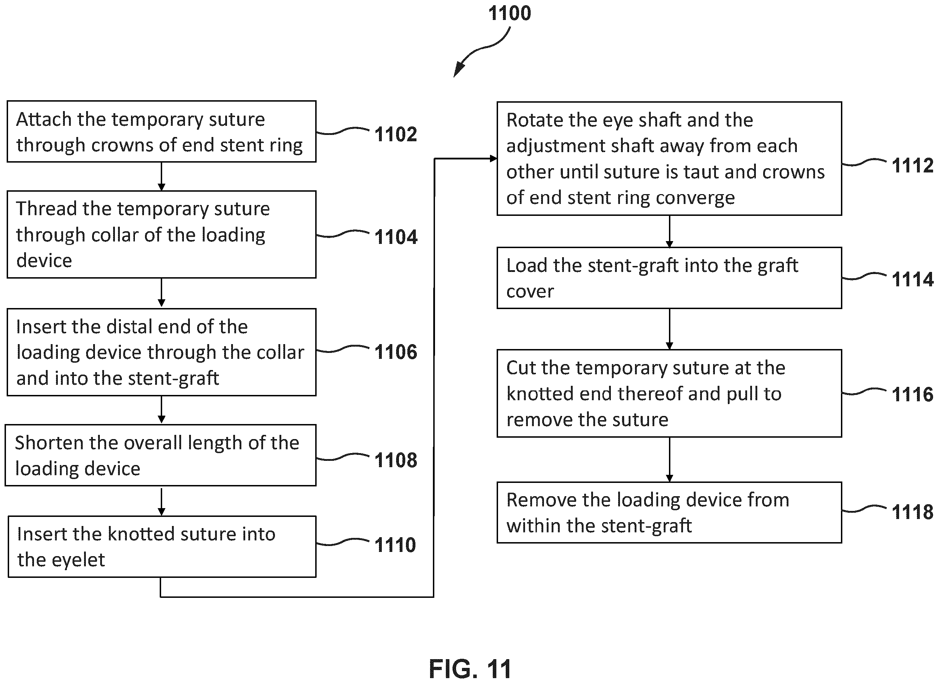

FIGS. 11-17D illustrate a method for loading a stent-graft into a graft cover with a loading device, in accordance with embodiments hereof. While described herein with the exemplary stent-graft 300 and the loading device 100, this is not meant to limiting, and other suitable stent-grafts and/or other embodiments of loading devices according to embodiments hereof may be utilized. FIG. 11 shows a flow chart with an overview of a method 1100 for loading the stent-graft 300 into a graft cover using the loading device 100. One skilled in the art will realize that FIGS. 11-17D illustrate one example of a method for loading a stent-graft into a graft cover using a loading device, and that steps of the method described may be removed and/or additional steps may be added. Additionally, the steps described need not be performed in the order described.

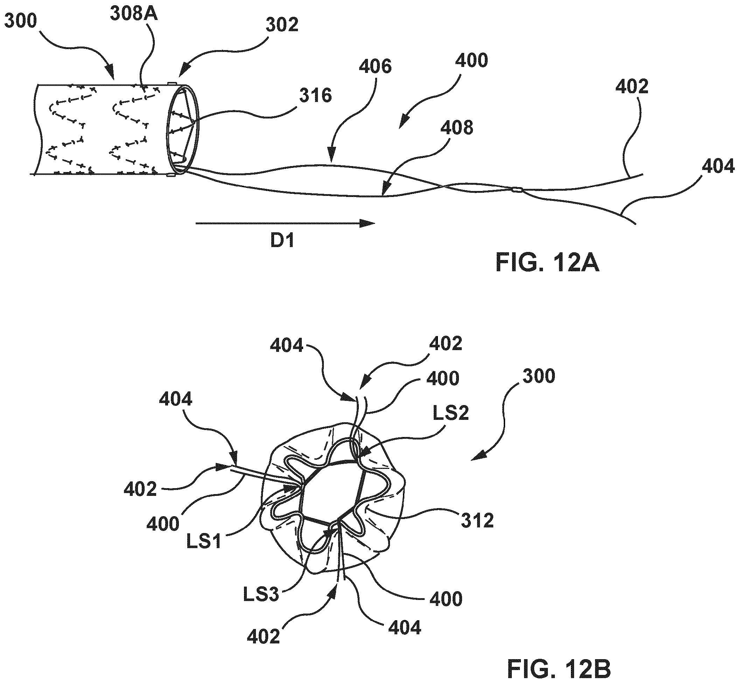

In a step 1102 of the method 1100, a suture 400 is coupled to the stent ring 308A at the first end 302 of the stent-graft 300. In an embodiment, a single suture 400 having a first end 402 and a second end 404 is coupled to the crowns 316 of the stent ring 308A that are adjacent the first end 302 of the stent-graft 300, as illustrated in FIG. 12A. More precisely, the first end 402 of the suture 400 is woven over a first crown 316 of the stent ring 308A adjacent the first end 302, *i.e.*, between the crown 316 and the inner surface 318 of the graft material 310. Suture 400 is then moved to the next adjacent crown 316 and is woven under/over the crown 316. This continues until the suture 400 is coupled to each crown 316 of first stent ring 308A and the suture 400 is circumferentially disposed substantially about the inner surface 318 of the graft material 310. Thus, the suture 400 circumscribes the inner surface 318 of the graft material 310, woven over and under the crowns 316 adjacent the first end 302 of the stent-graft 300. The first and second ends 402, 404 are aligned with each other, and the suture 400 is sufficiently long such that a first suture lead 406 is disposed between the first end 402 of the suture 400 and the first end 302 of the stent-graft 300, and a second suture lead 408 is disposed between the second end 404 of suture 400 and the first end 302 of the stent-graft 300, as shown in FIG. 12A. When a force is applied to suture 400 in a first direction D1, *i.e.* when the first and second ends 402, 404 are pulled away from the first end 302, the suture 400 brings the crowns 316 at the first end 302 of the stent-graft 300 closer to the central longitudinal axis of the stent-graft 300, thereby radially compressing the first end 302 of the stent-graft 300. The suture 400 may be of any suitable suture material, such as, but not limited to silk, nylon, polypropylene, polyester, polytetrafluoroethylene, or other materials suitable for the purposes described herein.

In the embodiment shown in FIG. 12A, there is a single suture 400. However, this is not meant to be limiting, and there may be more sutures 400, such as two sutures 400, or three sutures 400 or four sutures 400, or more sutures 400. For example, and not by way of limitation, in the embodiment of FIG. 12B, three sutures 400 are shown. Each suture 400 is woven over/under the crowns 316 at the first end 302 of the stent-graft 300 around the circumference of the inner surface 318 of the graft material 310, as explained above. However, the location where each suture 400 begins/ends the circumferential loop is radially spaced about circumference of the stent-graft 300. In other words, the location where each suture 400 transitions from its circumferential loop to the leads 406, 408 is spaced around the circumference of the stent-graft 300. For example, and not by way of limitation, the three sutures 400 shown in FIG. 12B begin/end the circumferential loop at locations LS1, LS2, and LS3, respectively, which are spaced about 120° apart from each other around the circumference of the stent-graft 300. The use of a plurality of the sutures 400 enables the compressive force applied by the sutures 400 to be more evenly distributed around the crowns 316 at the first end 302 of the stent-graft 300. The use of the plurality of sutures 400 also reduces the force required by each suture 400 to radially compress the first end 302 of the stent-graft 300.

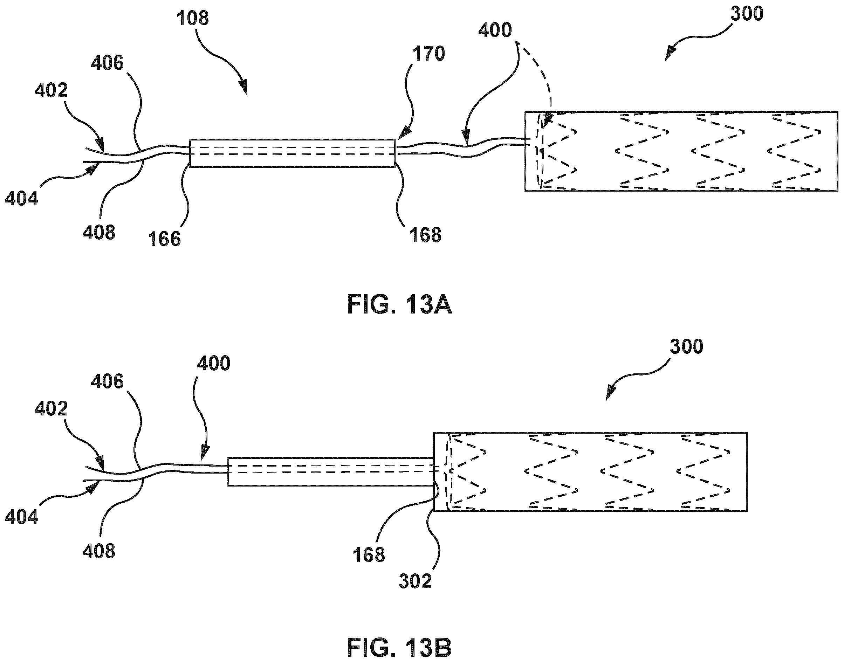

In another step 1104 of the method 1100, the suture 400 (or plurality of sutures 400) is threaded through the collar 108 of the loading device 100, as shown in FIGS. 13A-13B. In greater detail, the first and second ends 402, 404 of each suture 400, coupled to stent-graft 300 as previously described, are fed, guided, or advanced through the lumen 170 of the collar 108 from the distal end 168 to the proximal end 166 thereof. In an embodiment, the first and second leads 406, 408 may be advanced through lumen 170 until the first end 302 of stent-graft 300 is disposed adjacent the distal end 168 of collar 108, as illustrated in FIG. 13B. For clarity, FIGS. 13A-17D show a single suture 400. However, this is not meant to be limiting, and a plurality of sutures 400 may be used, as explained with respect to FIG. 12B.

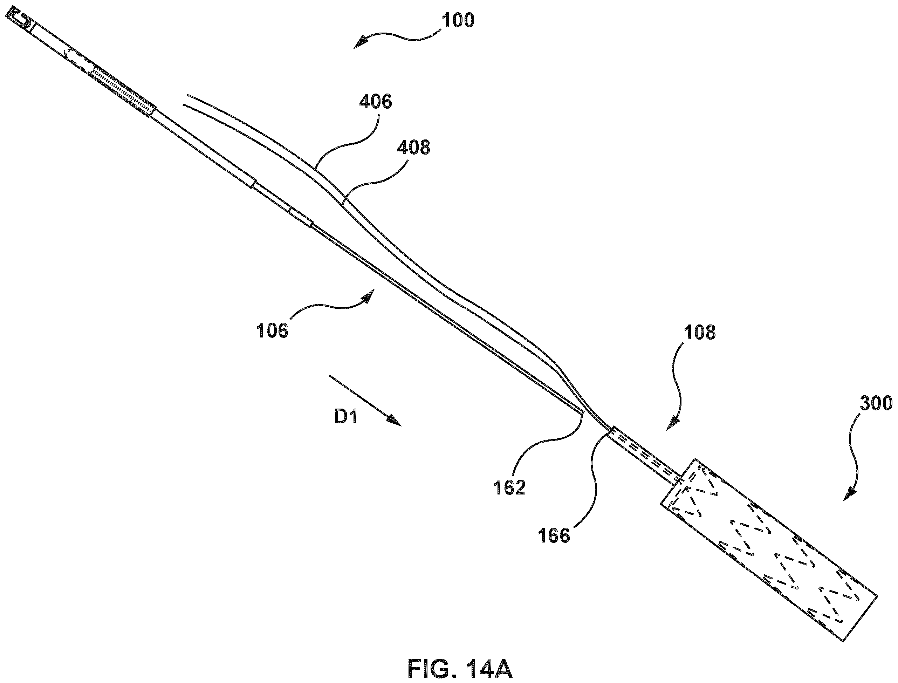

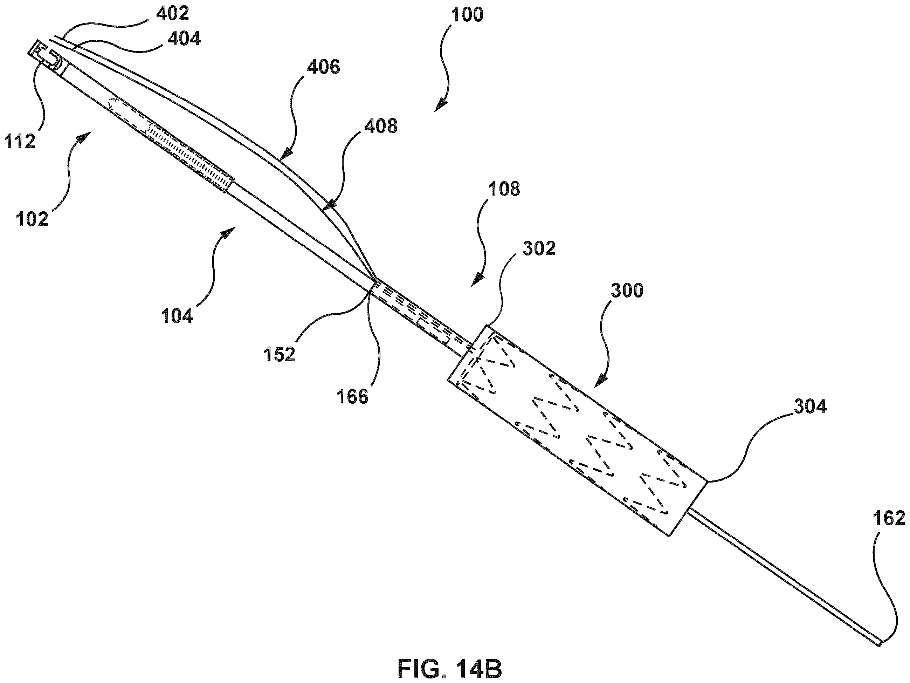

In another step 1106 of the method 1100, the distal end of the rod 106, coupled to the adjustment shaft 104 and the eye shaft 102, is inserted through the lumen 170 of the collar 108 from the proximal end 166 to the distal end thereof, as shown in FIG. 14A. The combined rod 106, adjustment shaft 104, and eye shaft 102 continues to be advanced in a first direction D1 such that the rod 106 extends through the collar 108 and through the stent-graft 300, as shown in FIG. 14B. Although described as the rod 106, the adjustment shaft 104, and the eye shaft 102 being advanced in the first direct D1, the collar 108 and stent-graft 300 could instead be translated in a second direction opposite the first direction D1, or the rod 106, the adjustment shaft 104, and the eye shaft 102 could be translated in the first direction D1 while the collar 108 and the stent-graft 300 are translated in the second direction. Thus, after the step 1106, the distal end 162 of the rod 106 is distal of the distal end 304 of the stent-graft 300, and the suture 400 is coupled to the first end 302 of the stent-graft 300 and extends proximally through the lumen 170 of the collar 108, exits the lumen 170 at the proximal end 166, and extends alongside the adjustment shaft 104 and the eye shaft 102 such that the first and second ends 402, 404 of the suture 400 are adjacent the eyelet 112, as shown in FIG. 14B. Further, the proximal end 166 of the collar 108 is disposed adjacent to the distal end 152 of center section 140 of adjustment shaft 104 of loading device 100, as shown in FIG. 14B.

In another step 1108 of the method 1100, if needed, the overall length of the loading device 100 is shortened such that the suture 400 can be looped through the eyelet 112. In particular, the adjustment shaft 104 and the eye shaft 102 are rotated relative to each other such that the threaded section 938 of the adjustment shaft 104 extend further into the cavity 914 of the eye shaft 102, *i.e.*, the proximal end 144 of the adjustment shaft 104 moves closer to the eyelet 112 of the eye shaft 102. The step 1108 can take place prior to or after any of the steps 1102 through 1106.

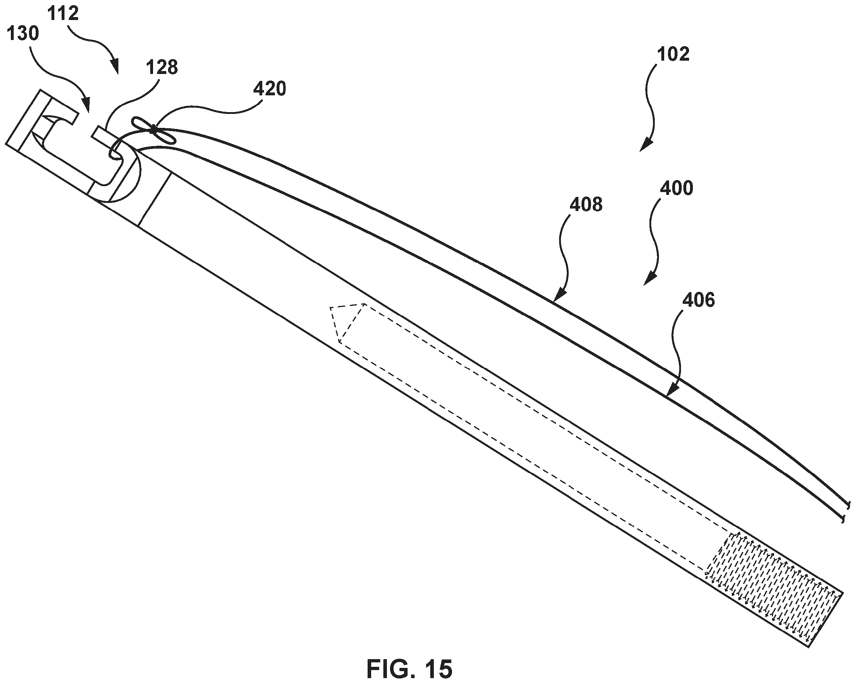

In another step 1110 of the method 1100, the suture 400 is coupled to the eyelet 112. In particular, the first and second leads 406, 408 may be coupled to each other such as by tying them together at a knot 420 adjacent the first and second ends 402, 404. The knotted suture 400 can be coupled to the eyelet 112 by passing the suture 400 through the gap 130 and into the eye 132, as shown in FIG. 15.

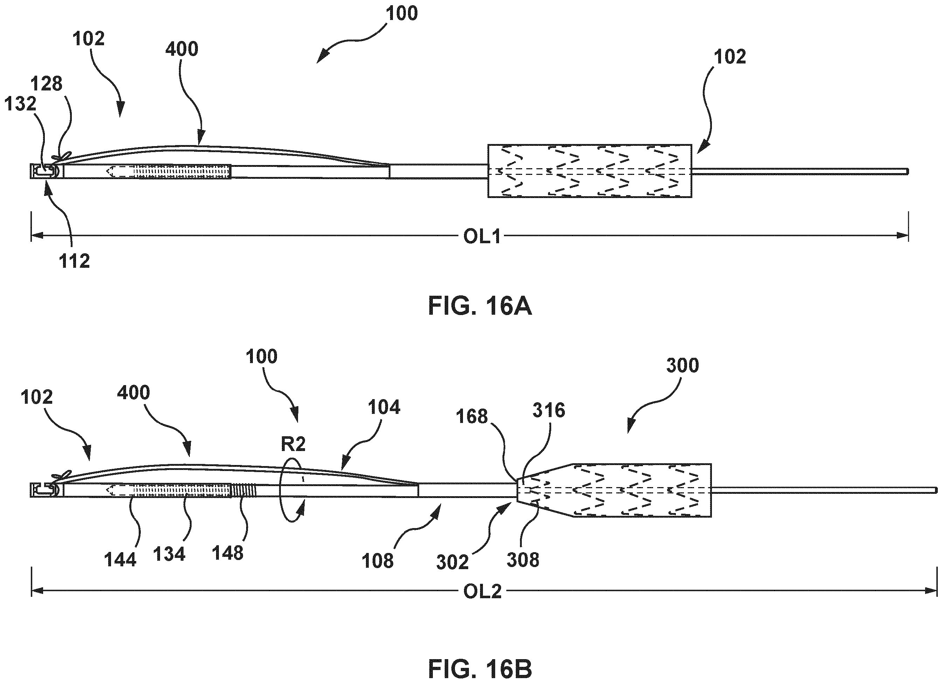

In another step 1112 of the method 1100, the adjustment shaft 104 and the eye shaft 102 are rotated relative to each other such that the threaded section 138 of the adjustment shaft 104 moves distally within the cavity 114 of the eye shaft 102. In an embodiment, the adjustment shaft 104 is rotated in the second direction R2, opposite first direction R1, such that second thread 148 of adjustment shaft 104 rotates relative to the first thread 134 in the cavity 114 such that the threaded section 138 moves distally within the cavity 114. The adjustment shaft 104 is preferably rotated (rather than the eye shaft 102) such that the plurality of sutures 400 do not radially wrap or twist about an outer surface of the loading device 100, but this is not meant to be limiting. As the threaded section 138, and hence the adjustment shaft 104, moves distally with respect to the eye shaft 102, the loading device 100 transitions from a first configuration with a first overall length OL1 (shown in FIG. 16A) to a second configuration with a second overall length OL2, wherein the second overall length OL2 is greater than the first overall length OL1 (shown in FIG. 16B). When the loading device 100 is lengthened to the second configuration with the second overall length OL2, tension is applied to the suture 400 such that the suture 400 pulls the crowns 316 at the first end 302 of the stent-graft 300 to transition the stent-graft 300 from the radially expanded configuration to the loading configuration. In greater detail, with the suture 400 coupled to eyelet 112, as the length of loading device 100 increases, tension is increased on the suture 400. The stent-graft 300 is held in place at the distal end 168 of the collar 108 by the suture 400 traveling through the lumen 170 of the collar 108 at the distal end 168. Therefore, the stent-graft 300 is unable to advance proximally relative to the loading device 100. Accordingly, when tension in first direction D1 is applied to the suture 400 by increasing the length of the loading device 100, the suture 400 applies radially compressive force on the crowns 316 of stent-graft 300 at the first end 302 thereof such that the stent-graft 300 transitions from the radially expanded configuration shown in FIG. 16A to the loading configuration shown in FIG. 16B. The adjustment shaft 104 is rotated in the second direction R2 until the crowns 316 of the stent ring 308A at the first end 302 of the stent-graft 300 converge, as shown in FIG. 16B.

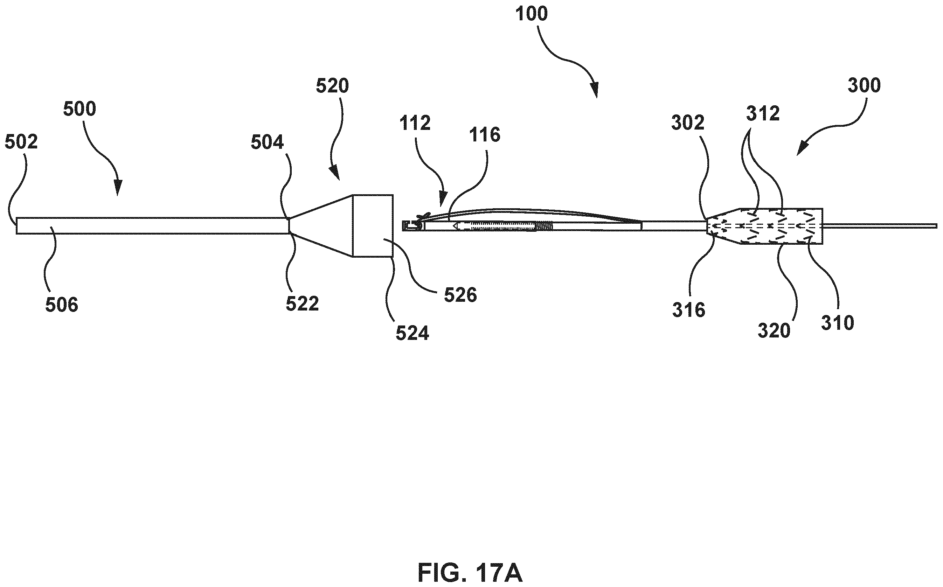

In another step 1114, the stent-graft 300 is loaded into a graft cover 500. In particular, and with reference to FIGS. 17A-17D, with the stent-graft 300 in the loading configuration and disposed on the loading device 100 as described above and shown in FIG. 16B, the stent-graft 300 is ready for loading into the graft cover 500. As shown in FIG. 17A, an exemplary graft cover 500 has a proximal end 502 and a distal end 504. A lumen 506 extends from the proximal end 502 to the distal end 504. The graft cover 500 may be any suitable graft cover corresponding for use with a delivery system for delivering a stent-graft. Further, in embodiments, the proximal end 502 of the graft cover 500 may be coupled to or continuous with an outer sheath of the delivery system. In an embodiment, a funnel 520 may also be used in assisting loading of the stent-graft 300 into the graft cover 500. The funnel 520 includes a proximal end 522, a distal end 524, and a lumen 526. The distal end 524 has a larger diameter than the proximal end 522 such that the lumen 526 tapers from the distal end 524 to the proximal end 522, thereby assisting in radially compressing the stent-graft 300. Those skilled in the art will recognize that the funnel 520 is not required, and may not be used or may be replaced with other components.

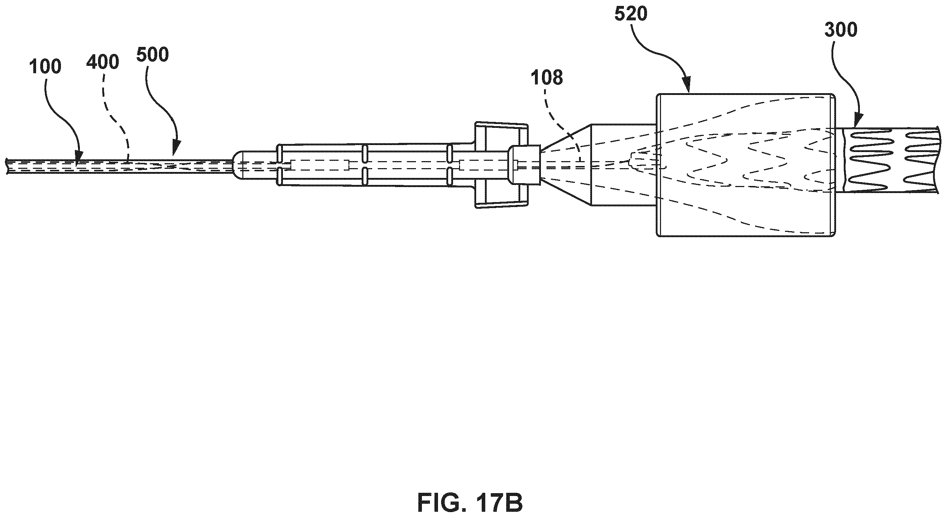

In the step 1114, a proximal section of loading device 100 is positioned within graft cover 500. More precisely, eyelet 112 is placed within the lumen 506 at the distal end 504 of the graft cover 500. As shown in FIGS. 17A and 17B, the loading device 100 is advanced in a first direction D1 through the graft cover 500 until the eyelet 112 is proximal of the proximal end 502 of the graft cover 500. The loading device 100 is then pulled proximally in the first direction such that the first end 302 of the stent-graft 300 and the crowns 316 of the stent ring 308A at the first end 302 of the stent-graft 300 are received within the lumen 506 of the graft cover 500. Holding the graft cover 500 firmly, the loading device 100, with the stent-graft 300 disposed thereon, continues to be pulled in the first direction D1, thereby radially compressing the stent-graft 300 as the stent-graft 300 is pulled into the graft cover 500. The loading device 100 is pulled through the lumen 506 of the graft cover 500 until the stent-graft 300 is fully disposed within the lumen 506. Those skilled in the art will recognize that instead of pulling the loading device 100 proximally through the graft cover 500 as described, the loading device 100 may be held firmly and the graft cover 500 (and the T-shaft 510 and the funnel 520) may be advanced relative to the loading device 100. The step 1114 may be accomplished manually or via a machine.

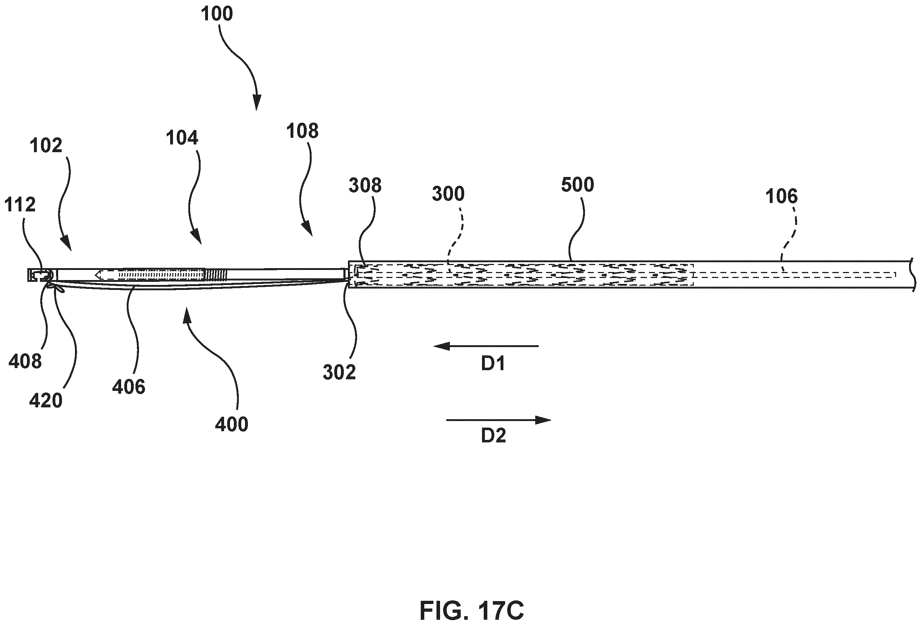

In another step 1116, with the stent-graft 300 in the radially compressed configuration disposed within the graft cover 500, and coupled to the loading device 100, as shown in FIG. 17C, the suture 400 is severed, preferably adjacent to the eye shaft 102. Thus, the first lead 406 and the second lead 408 are uncoupled from each other and from eyelet 112. The first end 402 of the suture 400 is pulled in the first direction D1, thereby pulling the first lead 406 in the first direction D1. As the first end 402 is pulled in the first direction D1, the second end 404 of the suture moves in the second direction D2 opposite the first direct D1, *i.e.,* towards the stent-graft 300. The second lead end 404 travels in second direction D2, along the eye shaft 102 and the adjustment shaft 104, and through the lumen 170 of the collar 108. Next, the second lead 408 travels circumferentially through the crowns 316 of the first stent ring 308A at the first end 302 of the stent 300, unweaving itself through the crowns 316 of the first stent ring 308A. Upon exiting the crowns 316 of the first stent ring 308A, the second lead 408 reverses direction and travels back in the first direction D1 through the lumen 170 of the collar 108 and is thereby removed or uncoupled from both the stent-graft 300 and the loading device 100.

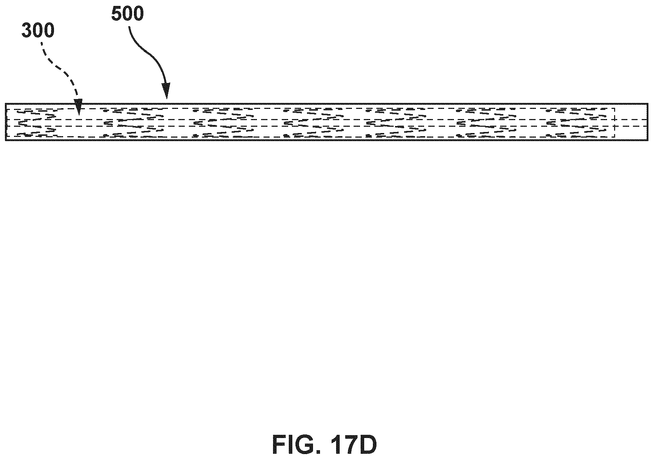

In another step 1118, the loading device 100 is advanced in the first direction D1 such that the loading device 100 exits the graft cover 500 and the stent-graft 300. The stent-graft 300 remains in the radially compressed configuration disposed within the lumen 506 of graft cover 500, as shown in FIG. 17D.

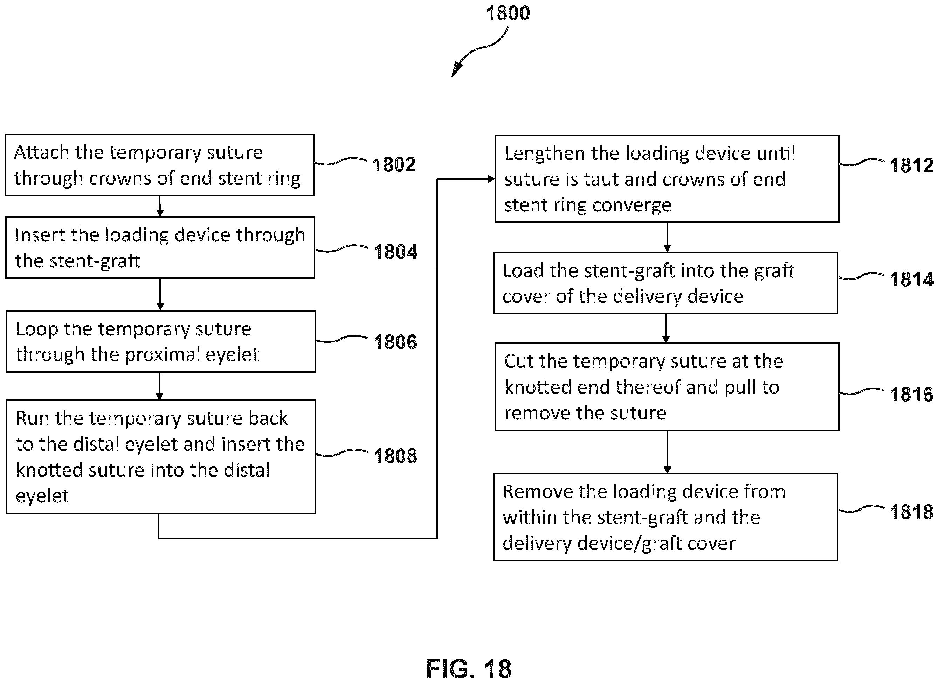

FIGS. 18-19K illustrate a method 1800 for loading a stent-graft into a graft cover of a delivery system, in accordance with embodiments hereof. The method described with respect to FIGS. 18-19K is particularly useful for re-loading a stent-graft into a delivery system at the geographic location of the procedure (e.g., a hospital). As explained briefly above, in some instances, as user at the geographic location of the procedure may need to remove the stent-graft from the delivery system (e.g., a graft cover of the delivery system) and then re-load the stent-graft back into the delivery system. However, this is not meant to be limiting. Further, the method of FIGS. 18-19K is described with respect to the loading device 200 described above. However, this is not meant to be limiting, the loading device 900 may be used for the method 1800. Further, described herein with the example stent-graft 300 and an example delivery device 600, this is not meant to limiting, and other suitable stent-grafts and/or other delivery devices may be utilized.

In a step 1802 of the method 1800, the suture or plurality of sutures 400 are threaded through the crowns 316 of the stent ring 308A at the first end 302 of the stent-graft 300. The step 1802 is as described above with respect to step 1102 and FIGS. 12A-12B, and therefore the details thereof are not repeated here.

In another step 1804 of the method 1800, the loading device 200 is inserted through the stent-graft 300. As noted above, the loading device 200 is symmetric in that the proximal and distal portions thereof both include adjustment shafts and eye shafts that are adjustable relative to each other to change the overall length of the loading device 200. Therefore, the direction that the loading device is inserted through the stent-graft 300 does not matter. If the loading device 900 is used, or a similar loading device, it is preferable that the proximal shaft 992 be disposed past the end of the stent-graft 300 that will be loaded into the graft cover of the delivery device 600 first. Thus, although the proximal shaft 992 is distal to the user operating the loading device 900, it is proximal with respect to the handle of the delivery device. Further, in some instances, based on the use of the stent-graft 300, the first end 302 may be the outflow end of the stent-graft 300.

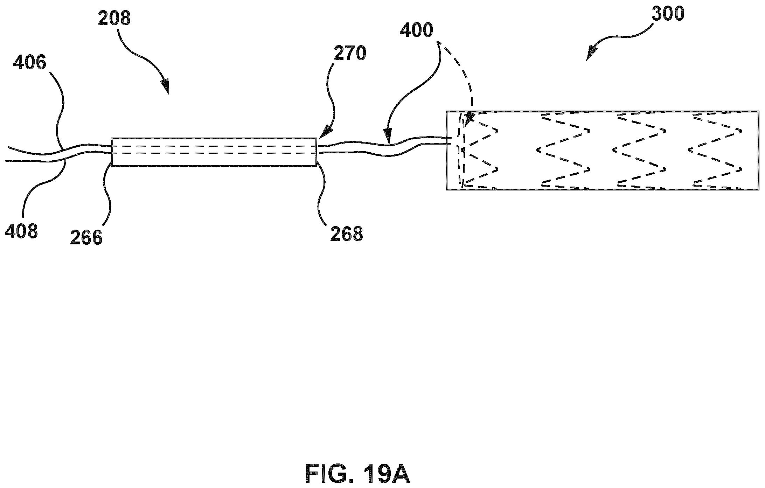

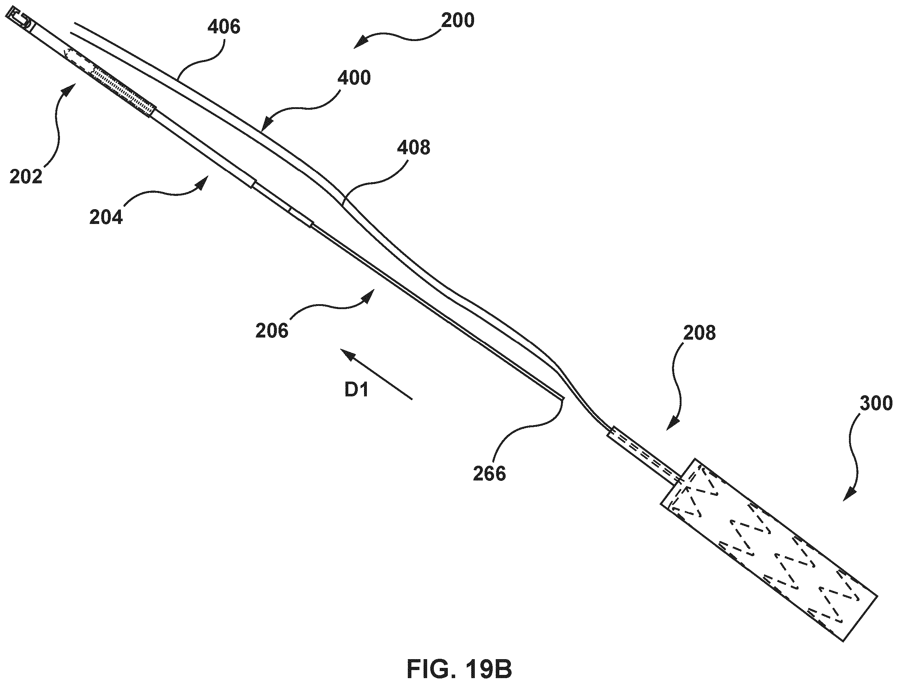

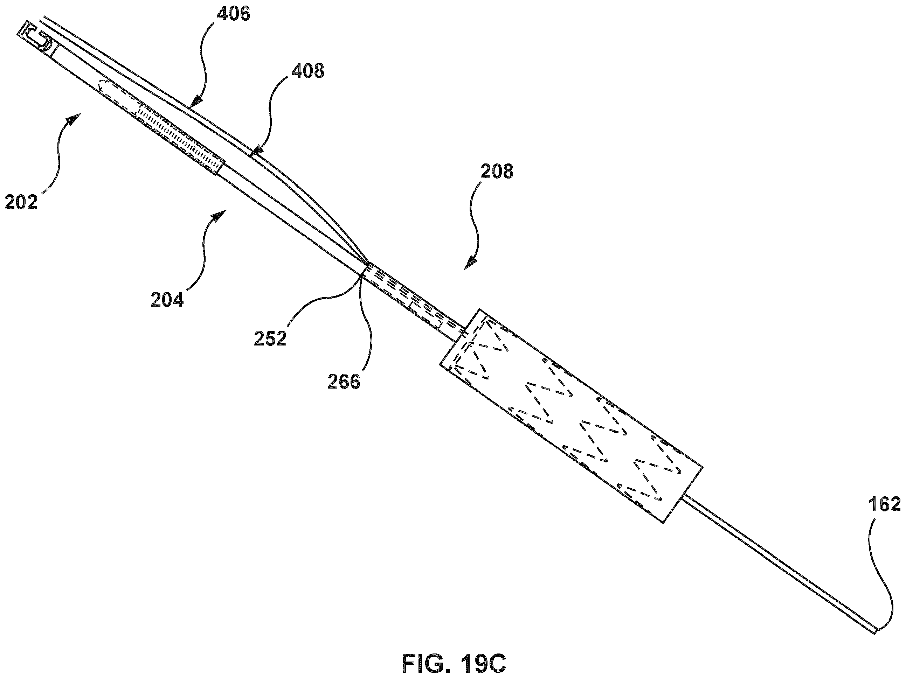

As part the step 1804, the suture 400 may be inserted through the proximal collar 208 of the loading device 200. The rod 206, with the proximal adjustment shaft 204 and the proximal eye shaft 202 coupled thereto, may then be inserted through the proximal collar 208 and the stent-graft 300, as shown in FIGS. 19B and 19C. The distal portion 266 of the rod 206 may then be coupled to the distal adjustment shaft 294 and the distal eye shaft 292. Similarly, if the loading device 900 is used, the suture 400 may be inserted through the proximal collar 908 of the loading device 900. The rod 906, with the shaft 902 coupled thereto, may then be inserted through the proximal collar 908 and the stent-graft 300. The distal portion 966 of the rod 906 may then be coupled to the distal adjustment shaft 904 and the distal eye shaft 902.

In another step 1806 of the method 1800, the suture 400 is looped through the proximal eyelet 212 of the proximal eye shaft 202. In particular, the leads 406, 408 of the suture 400 are translated in a first direction D1 (*i.e.,* from the first end 302 of the stent-graft 300 towards the proximal eye shaft 202 and inserted through the proximal eyelet 212, such as through the gap 130 and into the eye 132.

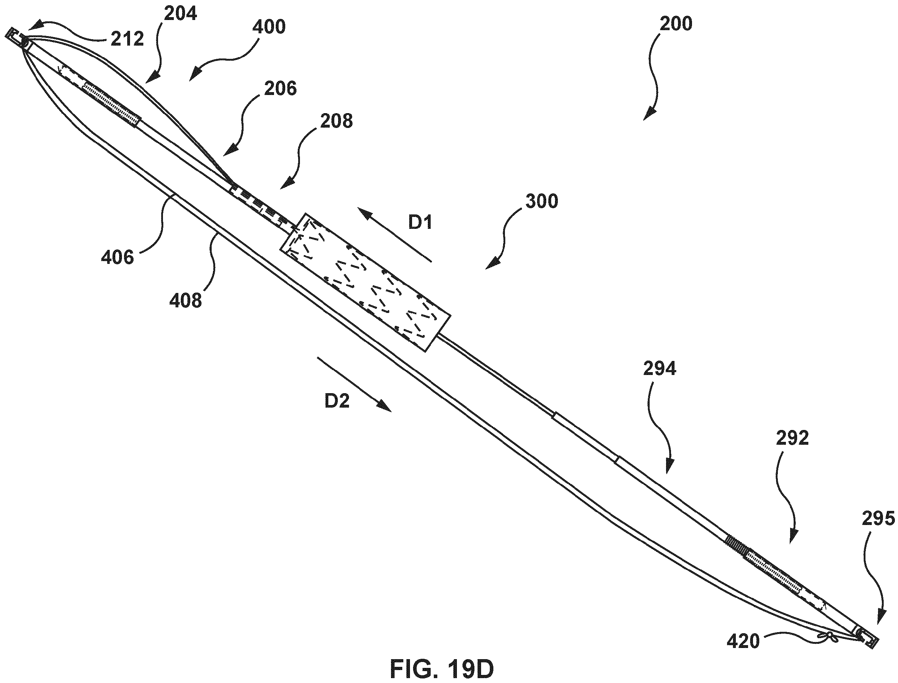

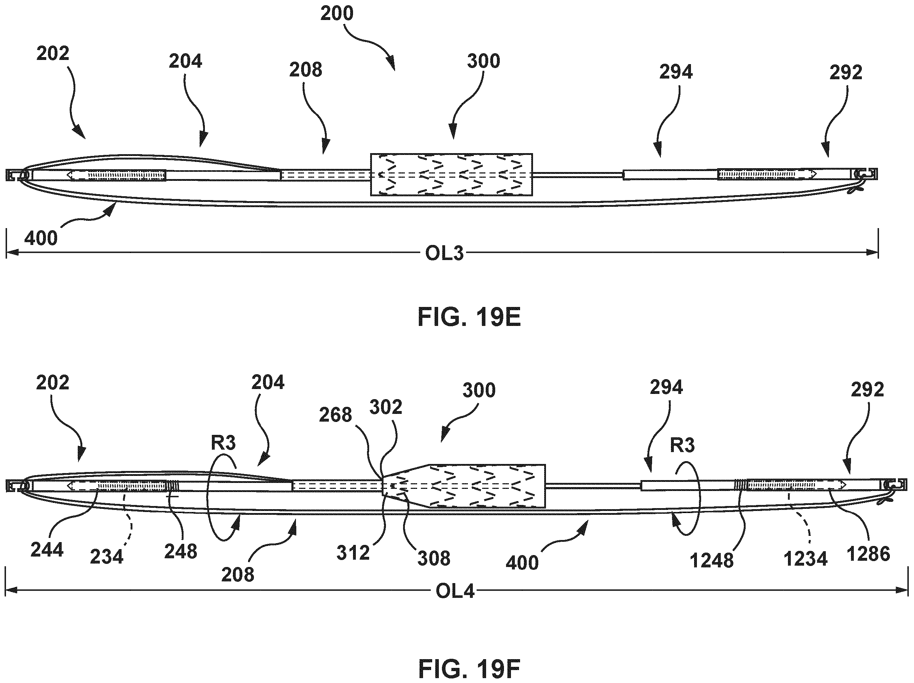

In another step 1808 of the method 1800, the suture 400 is extended back in a second direction D2 opposite the first direction D 1. In particular the suture leads 406, 408 are extended back in the second direction D2 from the proximal eyelet 212 towards the first end 302 of the stent-graft 300, past the stent-graft 300 (outside of the stent-graft 300) in the second direction D2, until the first and second ends 402, 404 of the suture 400 are disposed at the distal eyelet 295 of the distal eye shaft 292. The first and second ends 402, 404 of the suture 400 may be coupled together, such as at the knot 420, and inserted into the distal eyelet 295, such as through a corresponding gap of the eyelet 295, and into a corresponding eye of the eyelet 295. After the step 1808, the loading device 200 is coupled to the stent-graft 300 and the suture 400 is slack or loose, as shown in FIGS. 19D and 19E.

In another step 1810 of the method 1800, the loading device 200 is lengthened until the suture 400 is taut and the crowns 316 of the stent ring 308A at the first end 302 of the stent-graft 300 converge, as shown FIG. 19F. The loading device 200 may be lengthened by rotating the proximal adjustment shaft 204 relative to the proximal eye shaft 202 (for example in direction R3) and/or rotating the distal adjustment shaft 294 relative to the distal eye shaft 292 (for example in direction R3). Similarly, if the loading device 900 were used, the distal adjustment shaft 904 is rotated relative to the distal eye shaft 902 to lengthen the loading device 900. Rotation of the proximal adjustment shaft 204 and/or the distal adjustment shaft 294 (or the distal adjustment shaft 904 of the loading device 900) causes the proximal adjustment shaft 204 and the proximal eye shaft 202 and/or the distal adjustment shaft 294 and the distal eye shaft 292 (or the distal adjustment shaft 904 and the distal eye shaft 902) to separate due to the threaded engagement thereof, thereby causing the overall length of the loading device 200/900 to increase. As shown in comparing FIGS. 19E and 19F, the overall length of the loading device 200 increases from a third overall length OL3 to a fourth overall length OL4, wherein the fourth overall length OL4 is longer than the third overall length OL3. As explained above, the increased length of the loading device 200 causes the suture 400 to become taut, and at a particular length, apply a radially compressive force on the crowns 316 of the stent ring 308A at the first end 302 of the stent-graft 300, thereby causing the crowns 316 to radially compress or converge towards each other, as shown in FIG. 19F. In greater detail, with the suture 400 coupled to distal eyelet 212, 912, as the length of loading device 200, 900 increases, tension is increased on the suture 400. The stent-graft 300 is held in place by the collar 208, 908 by the suture 400 traveling through the lumen of the collar 208, 908. Therefore, the stent-graft 300 is unable to advance proximally relative to the loading device 200, 900. Accordingly, when tension is applied to the suture 400 by increasing the length of the loading device 200, 900,.the suture 400 applies radially compressive force on the crowns 316 of stent-graft 300 at the first end 302 thereof such that the stent-graft 300 transitions from the radially expanded configuration shown in FIG. 19E to the loading configuration shown in FIG. 19F.

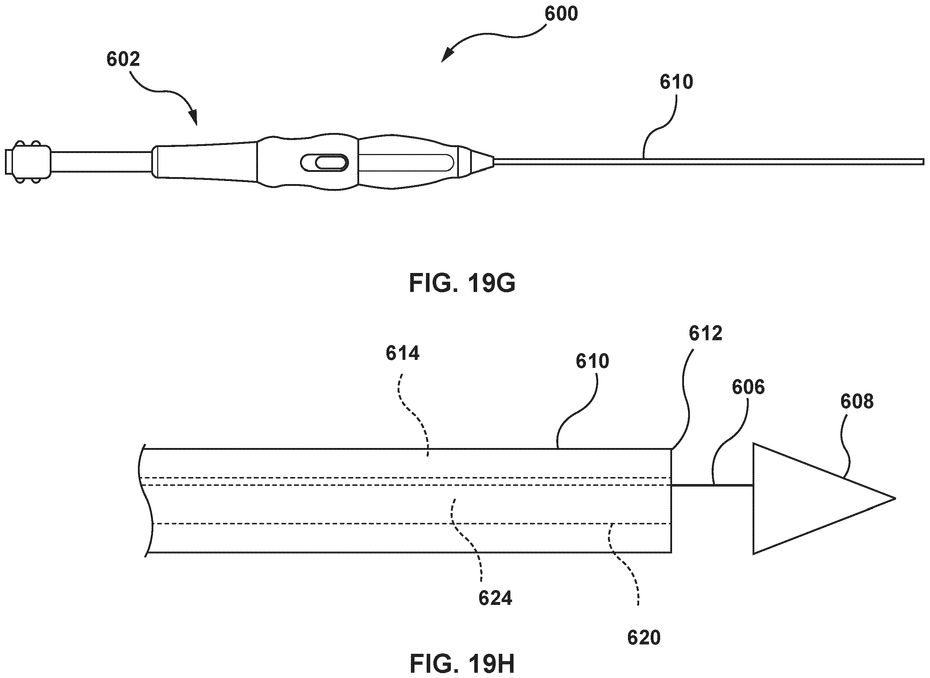

In another step 1814 of the method 1800, the stent-graft 300 is loaded into a graft cover of a delivery device. In particular, with the stent-graft 300 in the loading configuration and disposed on the loading device 200 as described above and shown in FIG. 19F, the stent-graft 300 is ready for loading into a graft cover 610 of a delivery device 600. FIGS. 19G and 19H show an example delivery device 600, including a handle 602 and a graft cover 610. The delivery device 600 may also include a stability shaft 620 including a stability lumen 624 extending therethrough. The delivery device 600 may also include a tip shaft 606 extending through the stability lumen 624, and a distal tip 608 coupled to the tip shaft 606, as shown in FIG. 19H. Those skilled in the art would recognize that the delivery device 600 includes other components and existing components may be added or removed from the delivery device 600. Further, the delivery device 600 is merely an example, and the loading devices described herein can be used with other delivery devices having a graft cover or similar item (e.g., an outer sheath or capsule). The graft cover 610 includes a distal end 612 and a lumen 614 extending through the graft cover 610. The lumen 614 is configured to receive the stent-graft 300 between the graft cover 610 and the stability shaft 620. The graft cover 610 may be any suitable graft cover suitable for delivery of the stent-graft 300.

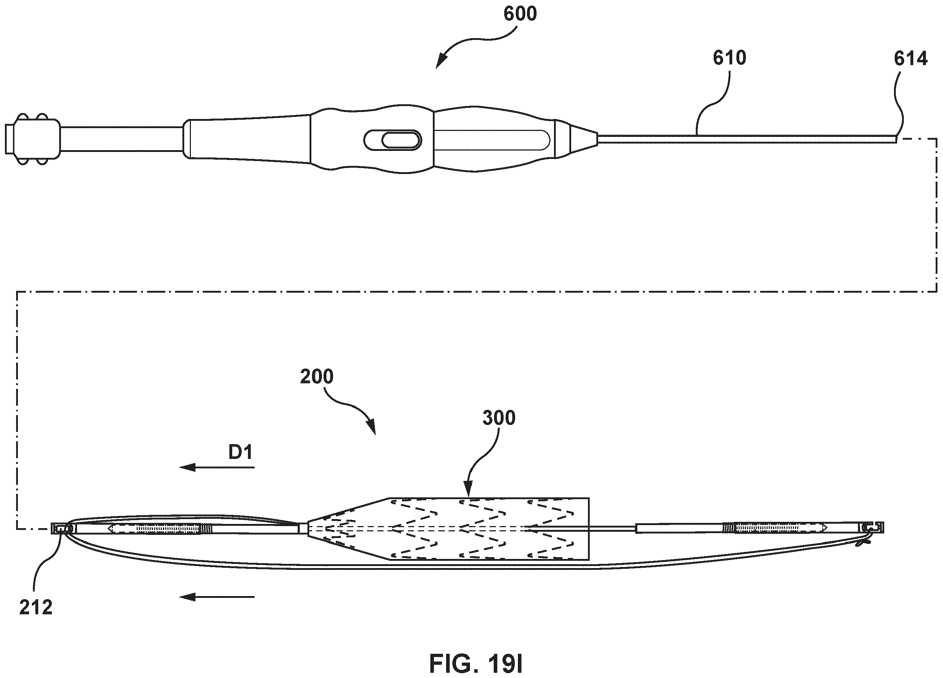

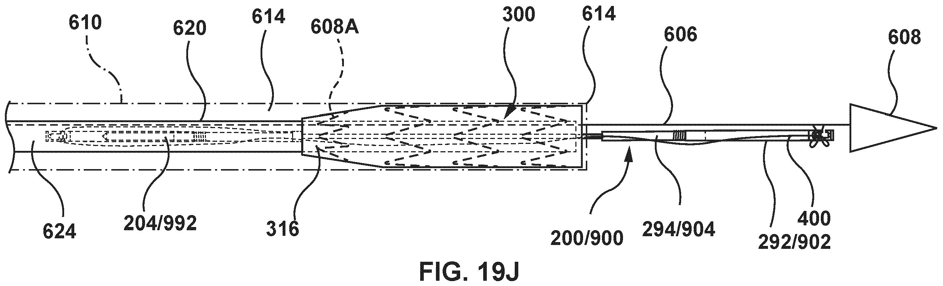

Thus, in the step 1814, the proximal eye shaft 202 or 992 of the loading device 200 or 900 is positioned adjacent the distal end 612 of the graft cover 610 of the delivery device 600, as shown in FIG. 19I. The loading device 200 or 900 in a first direction D1 (e.g., proximally) within the lumen 614 of the graft cover 610. As the loading device 200 or 900 and the stent-graft 300 is advanced proximally, the proximal portion of the loading device 200 or 900 (e.g., the proximal eye shaft 202/992 and the proximal adjustment shaft 204) is inserted into the stability lumen 624, but the stability shaft 620 is disposed through the stent-graft 300. Thus, the crowns 316 of the stent ring 308A at the first end 302 of the stent-graft 300 must enable the stability shaft 620 to extend therethrough, as shown in FIG. 19J. If necessary, the suture 400 may be loosened by adjusting the distal adjustment shaft 294 or 904 such that the crowns 316 separate sufficiently to enable the stability shaft 620 to enter into the lumen of the stent-graft 300, as shown in FIG. 19J.

In another step 1816 of the method 1800, with the stent-graft 300 in the radially compressed configuration disposed within the graft cover 610, and coupled to the loading device 200 or 900, as shown generally in FIG. 19J, the suture 400 is severed, preferably adjacent the distal eye shaft 292 or 902. Thus, the first lead 406 and the second lead 408 are uncoupled from each other and from the distal eyelet 295 or 912. The first end 402 of the suture 400 may then be pulled in a second direction (e.g., distally). The second end 404 of the suture 400 will thus be pulled in a first direction (e.g., proximally) along the distal eye shaft 292/902, along the distal adjustment shaft 294/904, along the outer surface of the stent-graft 300, along the proximal portion (202/204 or 992) of the loading device 200/900. The second end 404 is pulled through the proximal eyelet 212, 995 reversing direction. The second end 404 advances back in the second direction D2 towards the first end 302 of the stent-graft 300, is pulled through the crowns 316 at the first end 302 of the stent-graft 300. The second end 404 of the suture 400 then advances back in the first direction D1 towards the proximal eyelet 212, 995, is pulled through the proximal eyelet 212, 995, and extends back in the second direct D2 past the stent-graft 300 and the distal portion of the loading device 200, 900 until the second end 404 of the suture 400 is removed from the loading device 200, 900 and the delivery device 600.

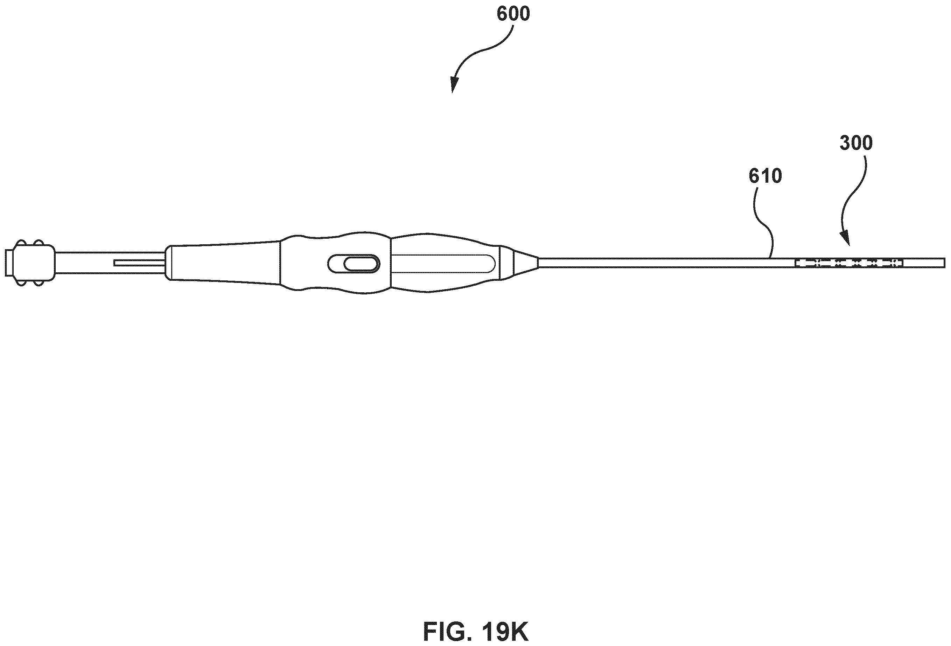

In another step 1818 of the method 1800, with the loading device 200, 900 no longer coupled to the stent-graft 300, the loading device 200, 900 can be removed from within the stent graft 300 and from within the delivery device 600. In particular, the loading device 200, 900 can be pulled in the second direction D2 to remove the loading device 200, 900 from within the stent-graft 300 and the graft cover 610 of the delivery device 600, leaving the stent graft 300 radially compressed within the lumen 614 of the graft cover 610, as shown in FIG. 19K.

Further disclosed herein a loading device for loading a graft-stent into a graft cover. The loading device includes an eye shaft, an adjustment shaft, a rod, and a collar. The eye shaft includes an eyelet disposed at a first end of the eye shaft. The adjustment shaft includes a first portion adjustably coupled to a second end of the eye shaft opposite the first end of the eye shaft such that an overall length of the loading device may be lengthened or shortened via the adjustment shaft. The rod is coupled to a second portion of the adjustment shaft opposite the first portion of the adjustment shaft. The collar is disposed over the rod and includes a collar lumen configured to receive a suture between an inner surface of the collar and an outer surface of the rod.

While only some embodiments have been described herein, it should be understood that the embodiments have been presented by way of illustration and example only, and not limitation. Various changes in form and detail can be made therein without departing from the spirit and scope of the invention, and each feature of the embodiment discussed herein, and of each reference cited herein, can be used in combination with the features of any other embodiment. All patents and publications discussed herein are incorporated by reference herein in their entirety.

Further disclosed herein is the subject matter of the following clauses:
1. A loading device for loading a graft-stent into a graft cover, the loading device comprising:
   an eye shaft including an eyelet disposed at a first end of the eye shaft;
   an adjustment shaft, wherein a first portion of the adjustment shaft is adjustably coupled to a second end of the eye shaft opposite the first end of the eye shaft such that an overall length of the loading device may be lengthened or shortened via the adjustment shaft; and
   a rod coupled to a second portion of the adjustment shaft opposite the first portion of the adjustment shaft.
2. The loading device of clause 1, further comprising a collar disposed over the rod, the collar including a collar lumen, wherein the collar lumen is configured to receive a suture between an inner surface of the collar and an outer surface of the rod.
3. The loading device of clause 1 or 2, wherein the eyelet includes a gap configured to receive a suture therethrough.
4. The loading device of clause 1 or of any preceding clause, wherein the second end of the eye shaft includes a first threaded portion and the first portion of the adjustment shaft includes a second threaded portion configured to engage the first threaded portion to adjustably couple the adjustment shaft and the eye shaft.
5. The loading device of clause 4, wherein the eye shaft includes a cavity at the second end, wherein the first threaded portion is disposed within the cavity, and wherein the first portion of the adjustment shaft is configured to be received within the cavity such that the second threaded portion engages the first threaded portion to adjustably couple the adjustment shaft and the eye shaft.
6. The loading device of clause 1 or of any preceding clause, wherein the second portion of the adjustment shaft includes a rod cavity, wherein the rod is configured to be disposed in the rod cavity to couple the rod to the adjustment shaft.
7. The loading device of clause 6, wherein the rod is configured to be press fit into the rod cavity to couple the rod to the adjustment shaft.
8. The loading device of clause 1 or of any preceding clause, further comprising:
   a stent-graft including a stent coupled to a graft material, the stent comprising a plurality of struts coupled to each other at crowns; and
   a suture,
   wherein the suture is configured to be threaded through a plurality of the crowns at a first end of the stent-graft and to the eyelet of the eye shaft, and
   wherein lengthening the overall length of the loading device via the adjustment shaft and the eye shaft is configured to make the suture taut and to radially converge the crowns at the first end of the stent-graft.
9. The loading device of clause 8, wherein a first end and a second end of the suture are coupled to each other adjacent the eyelet, and wherein a middle portion of the suture is threaded through the crowns at the first end of the stent.
10. The loading device of clause 8, wherein the stent comprises a plurality of stent rings, wherein the suture is coupled to the crowns of one of the plurality of stent rings at the first end of the stent-graft.
11. The loading device of clause 8, wherein the suture comprises a plurality of sutures.
12. A loading device for loading a graft-stent into a graft cover, the loading device comprising:
   a proximal shaft having a first eyelet disposed at a proximal end of the proximal shaft;
   a distal eye shaft including a second eyelet disposed at a distal end of the distal eye shaft;
   a distal adjustment shaft, wherein a distal portion of the distal adjustment shaft is adjustably coupled to a proximal end of the distal eye shaft opposite the distal end of the eye shaft such that an overall length of the loading device may be lengthened or shortened via the distal adjustment shaft; and
   a rod having a proximal portion coupled to a distal end of the proximal shaft and a distal portion coupled to a proximal portion of the distal adjustment shaft.
13. The loading device of clause 12, wherein the distal eyelet includes a gap configured to receive a suture therethrough.
14. The loading device of clause 12 or 13, wherein the proximal end of the eye shaft includes a first threaded portion and the distal portion of the adjustment shaft includes a second threaded portion configured to engage the first threaded portion to adjustably couple the distal adjustment shaft and the distal eye shaft.
15. The loading device of clause 14, wherein the distal eye shaft includes a cavity at the proximal end thereof, wherein the first threaded portion is disposed within the cavity, and wherein the distal portion of the adjustment shaft is configured to be received within the cavity such that the second threaded portion engages the first threaded portion to adjustably couple the distal adjustment shaft and the distal eye shaft.
16. The loading device of clause 12, wherein the proximal portion of the distal adjustment shaft includes a first rod cavity, wherein the distal portion of the rod is configured to be disposed in the first rod cavity to couple the rod to the distal adjustment shaft, and wherein the distal end of the proximal shaft include a second rod cavity, wherein the proximal portion of the rod is configured to be disposed in the second rod cavity to couple the rod to the proximal shaft.
17. The loading device of clause 16, wherein the distal portion of the rod is configured to be press fit into the first rod cavity to couple the rod to the distal adjustment shaft, and the proximal portion of the rod is configured to be press fit into the second rod cavity to couple the rod to the proximal shaft.
18. The loading device of clause 12, further comprising:
   a stent-graft including a stent coupled to a graft material, the stent comprising a plurality of struts coupled to each other at crowns; and
   a suture,
   wherein the suture is configured to be threaded through a plurality of the crowns at a proximal end of the stent-graft, extend proximally to the first eyelet, loop through the first eyelet, extend distally past the stent-graft and to the second eyelet at the distal end of the distal eye shaft, and
   wherein lengthening the overall length of the loading device via the distal adjustment shaft and the distal eye shaft is configured to make the suture taut and to radially converge the crowns at the proximal end of the stent.
19. The loading device of clause 18, wherein a first end and a second end of the suture are coupled to each other adjacent the second eyelet, and wherein a middle portion of the suture is threaded through the crowns at the proximal end of the stent-graft.
20. The loading device of clause 18, wherein the stent comprises a plurality of stent rings, wherein the suture is coupled to the crowns of one of the plurality of stent rings at the proximal end of the stent-graft.

## Claims

1. A loading device for loading a graft-stent into a graft cover, the loading device comprising:
an eye shaft including an eyelet disposed at a first end of the eye shaft;
an adjustment shaft, wherein a first portion of the adjustment shaft is adjustably coupled to a second end of the eye shaft opposite the first end of the eye shaft such that an overall length of the loading device may be lengthened or shortened via the adjustment shaft; and
a rod coupled to a second portion of the adjustment shaft opposite the first portion of the adjustment shaft.

2. The loading device of claim 1, further comprising a collar disposed over the rod, the collar including a collar lumen, wherein the collar lumen is configured to receive a suture between an inner surface of the collar and an outer surface of the rod.

3. The loading device of claim 1 or 2, wherein the eyelet includes a gap configured to receive a suture therethrough.

4. The loading device of any preceding claim, wherein the second end of the eye shaft includes a first threaded portion and the first portion of the adjustment shaft includes a second threaded portion configured to engage the first threaded portion to adjustably couple the adjustment shaft and the eye shaft; and optionally wherein the eye shaft includes a cavity at the second end, wherein the first threaded portion is disposed within the cavity, and wherein the first portion of the adjustment shaft is configured to be received within the cavity such that the second threaded portion engages the first threaded portion to adjustably couple the adjustment shaft and the eye shaft.

5. The loading device of any preceding claim, wherein the second portion of the adjustment shaft includes a rod cavity, wherein the rod is configured to be disposed in the rod cavity to couple the rod to the adjustment shaft; and optionally wherein the rod is configured to be press fit into the rod cavity to couple the rod to the adjustment shaft.

6. The loading device of any preceding claim, further comprising:
a stent-graft including a stent coupled to a graft material, the stent comprising a plurality of struts coupled to each other at crowns; and
a suture,
wherein the suture is configured to be threaded through a plurality of the crowns at a first end of the stent-graft and to the eyelet of the eye shaft, and
wherein lengthening the overall length of the loading device via the adjustment shaft and the eye shaft is configured to make the suture taut and to radially converge the crowns at the first end of the stent-graft.

7. The loading device of claim 6, wherein a first end and a second end of the suture are coupled to each other adjacent the eyelet, and wherein a middle portion of the suture is threaded through the crowns at the first end of the stent.

8. The loading device of claim 6 or 7, wherein the stent comprises a plurality of stent rings, wherein the suture is coupled to the crowns of one of the plurality of stent rings at the first end of the stent-graft; and/or wherein the suture comprises a plurality of sutures.

9. A loading device for loading a graft-stent into a graft cover, the loading device comprising:
a proximal shaft having a first eyelet disposed at a proximal end of the proximal shaft;
a distal eye shaft including a second eyelet disposed at a distal end of the distal eye shaft;
a distal adjustment shaft, wherein a distal portion of the distal adjustment shaft is adjustably coupled to a proximal end of the distal eye shaft opposite the distal end of the eye shaft such that an overall length of the loading device may be lengthened or shortened via the distal adjustment shaft; and
a rod having a proximal portion coupled to a distal end of the proximal shaft and a distal portion coupled to a proximal portion of the distal adjustment shaft.

10. The loading device of claim 9, wherein the distal eyelet includes a gap configured to receive a suture therethrough.

11. The loading device of claim 9 or 10, wherein the proximal end of the eye shaft includes a first threaded portion and the distal portion of the adjustment shaft includes a second threaded portion configured to engage the first threaded portion to adjustably couple the distal adjustment shaft and the distal eye shaft.

12. The loading device of claim 11, wherein the distal eye shaft includes a cavity at the proximal end thereof, wherein the first threaded portion is disposed within the cavity, and wherein the distal portion of the adjustment shaft is configured to be received within the cavity such that the second threaded portion engages the first threaded portion to adjustably couple the distal adjustment shaft and the distal eye shaft.

13. The loading device of any one of claims 9 to 12, wherein the proximal portion of the distal adjustment shaft includes a first rod cavity, wherein the distal portion of the rod is configured to be disposed in the first rod cavity to couple the rod to the distal adjustment shaft, and wherein the distal end of the proximal shaft include a second rod cavity, wherein the proximal portion of the rod is configured to be disposed in the second rod cavity to couple the rod to the proximal shaft; and optionally wherein the distal portion of the rod is configured to be press fit into the first rod cavity to couple the rod to the distal adjustment shaft, and the proximal portion of the rod is configured to be press fit into the second rod cavity to couple the rod to the proximal shaft.

14. The loading device of any one of claims 9 to 13, further comprising:
a stent-graft including a stent coupled to a graft material, the stent comprising a plurality of struts coupled to each other at crowns; and
a suture,
wherein the suture is configured to be threaded through a plurality of the crowns at a proximal end of the stent-graft, extend proximally to the first eyelet, loop through the first eyelet, extend distally past the stent-graft and to the second eyelet at the distal end of the distal eye shaft, and
wherein lengthening the overall length of the loading device via the distal adjustment shaft and the distal eye shaft is configured to make the suture taut and to radially converge the crowns at the proximal end of the stent.

15. The loading device of claim 14, wherein a first end and a second end of the suture are coupled to each other adjacent the second eyelet, and wherein a middle portion of the suture is threaded through the crowns at the proximal end of the stent-graft; and/or wherein the stent comprises a plurality of stent rings, wherein the suture is coupled to the crowns of one of the plurality of stent rings at the proximal end of the stent-graft.
